# EUROPEAN PATENT APPLICATION

(11) **EP 4 224 484 A1**
(43) Date of publication of application: **09.08.2023**
(21) Application number: 20957752.7
(22) Date of filing: 16.10.2020
(51) Int. Cl.: G16H 20/30, G16H 20/70, G16H 80/00

(54) **SERVER DEVICE, TREATMENT SUPPORT SYSTEM, TREATMENT SUPPORT METHOD, AND PROGRAM**

(71) Applicant: Life Quest Inc., Tokyo 107-0062 (JP)
(72) Inventor: KAYAMA, Tetsu, Tokyo 107-0062 (JP); SAITO, Ryozo, Tokyo 107-0062 (JP); TAKAHASHI, Hirotsugu, Tokyo 107-0062 (JP); FUKUDA, Takuma, Tokyo 107-0062 (JP); HAMAGUCHI, Reo, Tokyo 107-0062 (JP)
(74) Representative: Biesse S.r.l.
(86) International application number: PCT/JP2020/039197
(87) International publication number: WO 2022/079923

(57) **Abstract**

The present invention prevents worsening of symptoms of a patient. A server device (4) is provided with: a message database connected via a network N to a patient terminal (2) used by a patient for training for a treatment, and to a supporter terminal (3-n) of a supporter of the patient, the message database registering messages constructive to the treatment of the patient; and a control unit which, when a predetermined condition is satisfied, causes the messages registered in the message database to be displayed on the supporter terminal (3-n) in a selectable manner via the network (N), and causes a message selected by the supporter on the supporter terminal (3-n) to be displayed on the patient terminal (2) via the network (N) as a message from the supporter.

## Description

### TECHNICAL FIELD

The present invention relates to a server device, a treatment support system, a treatment support method, and a program, and more particularly, to a server device, a treatment support system, a treatment support method, and a program capable of preventing a deterioration of symptoms of a patient.

### BACKGROUND

As a group therapy for children with developmental disorders such as autism spectrum syndrome and their guardians, a program called "parent training" developed in the United States in the 1960s is widely known, and has achieved a certain level of success. This "parent training" enables the guardians to acquire knowledge of the way of contacting the children with the developmental disorders, the way of feedback, and the like (see, for example, Patent Document 1). Note that the specification, claims, and drawings of Patent Document 1 are incorporated herein by reference in their entirety.

### PRIOR ART DOCUMENT

### PATENT DOCUMENT

Patent Document 1: JP 2005-215019 A

### SUMMARY OF THE INVENTION

### PROBLEMS TO BE SOLVED BY THE INVENTION

Such a group therapy may also be beneficial in patients with Mild Cognitive Impairment (MCI) or dementia. However, the patients with MCI have symptoms such as memory impairment and disorientation, and unstable emotional expressions such as depression and agitation. For this reason, they may become emotional or deeply hurt by mistaking the meaning of messages from their supporters, thereby losing patient's motivation for training and, on the contrary, worsening the symptoms of the patients with MCI. In this way, it is not easy for the neighboring supporters to smoothly support the patients with MCI or dementia. Thus, it is necessary to pay close attention and have appropriate knowledge for the feedback from the neighboring supporters to the patients with MCI or dementia.

The present invention has been made to solve the above problems, and an object of the present invention is to provide a server device, a treatment support system, a treatment support method, and a program capable of preventing a deterioration of symptoms of each of the patients.

### MEANS FOR SOLVING THE PROBLEMS

In order to achieve the above object, a server device (4) according to a first aspect of the present invention is connected to a patient terminal (2) used by a patient for training for treatment and a supporter terminal (3-n) of a supporter of the patient via a network (N). The server device (4) includes:
a message database (422) which registers messages effective for the treatment of the patient; and
a server control unit (43) wherein when a predetermined condition is satisfied, the server control unit (43) causes the messages registered in the message database (422) to be displayed on the supporter terminal (3-n) via the network (N) in a selectable manner, and wherein the server control unit (43) causes a message selected by the supporter with the supporter terminal (3-n) to be displayed on the patient terminal (2) via the network (N) as a message from the supporter.

In the above-described server device (4), in response to receiving a usage history of the patient terminal (2) via the network (N), the server control unit (43) may determine whether or not a disengagement sign from the training is recognized in the patient based on the usage history of the patient terminal (2), and
if the server control unit (43) determines that the disengagement sign is recognized in the patient, assuming that the predetermined condition is satisfied, the server control unit (43) causes the messages registered in the message database (422) to be displayed on the supporter terminal (3-n) via the network (N) in a selectable manner.

In the above-described server device (4), the server control unit (43) may specify a frequency of use of a treatment program for treating the patient based on the usage history of the patient terminal (2), the treatment program installed in the patient terminal (2), and
if the frequency of use of the treatment program does not satisfy a predetermined standard, the server control unit (43) determines that the disengagement sign from the training is recognized in the patient.

In the above-described server device (4), the server control unit (43) may specify a degree of achievement of a treatment menu of the patient based on the usage history of the patient terminal (2), and
if the degree of achievement of the treatment menu does not satisfy a predetermined standard, the server control unit (43) determines that the disengagement sign from the training is recognized in the patient.

In the above-described server device (4), if the frequency of use of the treatment program satisfies the predetermined standard, the server control unit (43) may specify a degree of achievement of a treatment menu of the patient based on the usage history of the patient terminal (2), and
if the frequency of use of the treatment program satisfies the predetermined standard, but the degree of achievement of the treatment menu does not satisfy the predetermined standard, the server control unit (43) determines that the disengagement sign from the training is recognized in the patient.

In the above-described server device (4), in response to receiving an answer to a questionnaire about a lifestyle habit via the network (N), the answer input by the patient into the patient terminal (2), the server control unit (43) may determine whether or not a disturbance of the lifestyle habit is recognized in the patient from the answer to the questionnaire about the lifestyle habit, and
if the server control unit (43) determines that the disturbance of the lifestyle habit is recognized in the patient, assuming that the predetermined condition is satisfied, the server control unit (43) causes the messages registered in the message database (422) to be displayed on the supporter terminal (3-n) via the network (N) in a selectable manner.

In the above-described server device (4), in response to receiving a usage history of the patient terminal (2) via the network (N), the server control unit (43) may determine whether or not a disengagement sign from the training is recognized in the patient based on the usage history of the patient terminal (2), and
if the server control unit (43) determines that the disengagement sign is recognized in the patient, the server control unit (43) determines whether or not the disturbance of the lifestyle habit is recognized therein.

In the above-described server device (4), in response to receiving an answer to a questionnaire about a patient's mood via the network (N), the answer input by the patient into the patient terminal (2), the server control unit (43) may determine whether or not a deterioration of a mental state is recognized in the patient from a viewpoint of the patient based on the answer to the questionnaire about the patient's mood, and
if the server control unit (43) determines that the deterioration of the mental state is recognized in the patient from the viewpoint of the patient, assuming that the predetermined condition is satisfied, the server control unit (43) causes the messages registered in the message database (422) to be displayed on the supporter terminal (3-n) via the network (N) in a selectable manner.

In the above-described server device (4), in response to receiving an answer to a questionnaire about the patient's mood via the network (N), the answer input by the supporter into the supporter terminal (3-n), the server control unit (43) may determine whether or not the deterioration of the mental state is recognized in the patient from a viewpoint of the supporter based on the answer to the questionnaire about the patient's mood, and
if the server control unit (43) determines that the deterioration of the mental state is recognized in the patient from the viewpoint of the patient or the viewpoint of the supporter, assuming that the predetermined condition is satisfied, the server control unit (43) causes the messages registered in the message database (422) to be displayed on the supporter terminal (3-n) via the network (N) in a selectable manner.

In the above-described server device (4), in response to receiving a usage history of the patient terminal (2) and an answer to a questionnaire about a lifestyle habit via the network (N), the answer input by the patient into the patient terminal (2), the server control unit (43) may specify a degree of achievement of a treatment menu of the patient based on the usage history of the patient terminal (2), and specify a degree of achievement of the lifestyle habit of the patient from the answer to the questionnaire about the lifestyle habit, and
if both the treatment menu and the lifestyle habit satisfy a predetermined standard, the server control unit (43) causes messages praising the patient among the messages registered in the message database (422) to be displayed on the supporter terminal (3-n) via the network (N) in a selectable mannered, or if the treatment menu or the lifestyle habit does not satisfy the predetermined standard, the server control unit (43) causes messages for supporting the patient among the messages registered in the message database (422) to be displayed on the supporter terminal (3-n) via the network (N) in a selectable manner.

In the above-described server device (4), in response to receiving an answer to a questionnaire about a treatment activity via the network (N), the answer input by the patient into the patient terminal (2), the server control unit (43) may determine whether or not a disengagement sign from the training is recognized in the patient from the answer to the questionnaire about the treatment activity, and
if the server control unit (43) determines that the disengagement sign is recognized in the patient, assuming that the predetermined condition is satisfied, the server control unit (43) causes the messages registered in the message database (422) to be displayed on the supporter terminal (3-n) via the network (N) in a selectable manner.

A treatment support system (1) according to a second aspect of the present invention includes: a server device (4) connected to a patient terminal (2) used by a patient for training for treatment via a network (N); and a supporter terminal (3-n) of a supporter of the patient connected to the server device (4) via the network (N),
wherein the server device (4) includes:
a message database (422) which registers messages effective for the treatment of the patient; and
a server control unit (43) wherein when a predetermined condition is satisfied, the server control unit (43) transmits display data for displaying the messages registered in the message database (422) in a selectable manner to the supporter terminal (3-n) via the network (N), and wherein in response to receiving a message selection command transmitted from the supporter terminal (3-n) via the network (N), the server control unit (43) causes a message specified from the message selection command to be displayed on the patient terminal (2) via the network (N) as a message from the supporter,
wherein the supporter terminal (3-n) includes:
   a display unit (32) wherein in response to receiving the display data transmitted from the server device (4) via the network (N), the display unit (32) displays the messages effective for the treatment of the patient in a selectable manner based on the display data; and
   a terminal control unit (34) which transmits the message selection command indicating a message selected by the supporter from among the messages displayed on the display unit (32) to the server device (4) via the network (N).

A treatment support method according to a third aspect of the present invention is performed by a server device (4) connected to a patient terminal (2) used by a patient for training for treatment and a supporter terminal (3-n) of a supporter of the patient via a network (N), the server device (4) having a message database (422) for registering messages effective for the treatment of the patient, the treatment support method including:
displaying the messages registered in the message database (422) on the supporter terminal (3-n) via the network (N) in a selectable manner, when a predetermined condition is satisfied; and
displaying a message selected by the supporter with the supporter terminal (3-n) on the patient terminal (2) via the network (N) as a message from the supporter.

A treatment support method according to a fourth aspect of the present invention is performed in a treatment support system (1) including: a server device (4) connected to a patient terminal (2) used by a patient for training for treatment via a network (N) and having a message database (422) for registering messages effective for the treatment of the patient; and a supporter terminal (3-n) of a supporter of the patient connected to the server device (4) via the network (N),
wherein when a predetermined condition is satisfied, the server device (4) transmits display data for displaying the messages registered in the message database (422) in a selectable manner to the supporter terminal (3-n) via the network (N),
wherein in response to receiving the display data transmitted from the server device (4) via the network (N), the supporter terminal (3-n) displays the messages registered in the message database (422) on a display unit (32) in a selectable manner based on the display data,
wherein the supporter terminal (3-n) transmits a message selection command indicating a message selected by the supporter from among the messages displayed on the display unit (32) to the server device (4) via the network (N), and
wherein in response to receiving the message selection command transmitted from the supporter terminal (3-n) via the network (N), the server device (4) causes the message specified from the message selection command to be displayed on the patient terminal (2) via the network (N) as a message from the supporter.

A program according to a fifth aspect of the present invention causes a computer of a server device (4), which is connected to a patient terminal (2) used by a patient for training for treatment and a supporter terminal (3-n) of a supporter of the patient via a network (N), and includes a message database (422) for registering messages effective for the treatment of the patient, to execute
a process for displaying the messages registered in the message database (422) on the supporter terminal (3-n) via the network (N) in a selectable manner, when a predetermined condition is satisfied; and
a process for displaying a message selected by the supporter with the supporter terminal (3-n) on the patient terminal (2) via the network (N) as a message from the supporter.

A supporter terminal (3-n) according to a sixth aspect of the present invention includes:
a display unit (32) for displaying messages effective for treatment of a patient in a selectable manner, the messages prepared in advance, when being notified that the patient starts training for the treatment using a patient terminal (2) via a network (N); and
a terminal control unit (34) for causing a message selected by a supporter of the patient from among the messages displayed on the display unit (32) to be displayed on the patient terminal (2) via the network (N) as a message from the supporter.

In the above-described supporter terminal (3-n), it is preferable that the display unit (32) displays the messages corresponding to characteristics of the patient in a selectable manner.

In the above-described supporter terminal (3-n), in response to an indication of the supporter to participate in a training support for the patient, the display unit (32) may display the messages in a selectable manner after being notified of the start of the training of the patient.

In the above-described supporter terminal (3-n), it is preferable that the display unit (32) displays a state of the training of the patient.

A server device (4) according to a seventh aspect of the present invention is connected to a patient terminal (2) used by a patient for training for treatment and a supporter terminal (3-n) of a supporter of the patient via a network (N). The server device (4) includes:
a message database (422) which registers messages effective for the treatment of the patient; and
a server control unit (43) wherein in response to a notification from the patient terminal (2) via the network (N) of a start of the training of the patient, the server control unit (43) causes the messages registered in the message database (422) to be displayed on the supporter terminal (3-n) via the network (N) in a selectable manner, and wherein the server control unit (43) causes a message selected by the supporter with the supporter terminal (3-n) to be displayed on the patient terminal (2) via the network (N) as a message from the supporter.

Preferably, the above-described server device (4) further includes a patient database (421) for registering patient's characteristics in association with each patient,
wherein the message database (422) registers the messages in association with the patient's characteristics, and
wherein in response to the notification from the patient terminal (2) via the network (N) of the start of the training of the patient, the server control unit (43) reads (reads out) the patient's characteristics from the patient database (421), detects messages corresponding to the patient's characteristics from the message database (422), and causes the messages to be displayed on the supporter terminal (3-n) via the network (N) in a selectable manner.

The above-described server device (4) may further include a patient database (421) in which supporter information capable of identifying the supporter terminal (3-n) of the supporter of the patient is registered in association with each patient,
wherein in response to the notification from the patient terminal (2) via the network (N) of the start of the training of the patient, the server control unit (43) reads the supporter information of the patient from the patient database (421), and causes the messages registered in the message database (422) to be displayed on the supporter terminal (3-n) via the network (N) in a selectable manner, the supporter terminal (3-n) identified from the supporter information read from the patient database (421).

In the above-described server device (4), in response to a notification from the patient terminal (2) via the network (N) of the start of the training of the patient, the server control unit (43) further notifies the start of the training of the patient to the supporter terminal (3-n) of the supporter of the patient via the network (N), and
in response to an indication from the supporter terminal (3-n) to participate in a training support for the patient via the network (N), the server control unit (43) causes the messages registered in the message database (422) to be displayed on the supporter terminal (3-n) indicating to participate in the training for the patient via the network (N) in a selectable manner.

A treatment support system (1) according to an eighth aspect of the present invention includes: a server device (4) connected to a patient terminal (2) used by a patient for training for treatment via a network (N); and a supporter terminal (3-n) of a supporter of the patient connected to the server device (4) via the network (N),
wherein the server device (4) includes:
a message database (422) which registers messages effective for the treatment of the patient; and
a server control unit (43) wherein in response to a notification from the patient terminal (2) via the network (N) of a start of the training of the patient, the server control unit (43) transmits display data for displaying the messages registered in the message database (422) in a selectable manner to the supporter terminal (3-n) via the network (N), and wherein in response to receiving a message selection command transmitted from the supporter terminal (3-n) via the network (N), the server control unit (43) causes a message specified from the message selection command to be displayed on the patient terminal (2) via the network (N) as a message from the supporter,
wherein the supporter terminal (3-n) includes:
   a display unit (32) wherein in response to receiving the display data transmitted from the server device (4) via the network (N), the display unit (32) displays the messages registered in the message database (422) in a selectable manner based on the display data; and
   a terminal control unit (34) which transmits the message selection command indicating a message selected by the supporter from among the messages displayed on the display unit (32) to the server device (4) via the network (N).

A treatment support method according to a ninth aspect of the present invention includes:
displaying messages effective for treatment of a patient in a selectable manner, the messages prepared in advance, when being notified that the patient starts training for the treatment using a patient terminal (2) via a network (N); and
causing a message selected by a supporter of the patient from among messages displayed on a display unit (32) to be displayed on the patient terminal (2) via the network (N) as a message from the supporter.

A treatment support method according to a tenth aspect of the present invention is performed by a server device (4) connected to a patient terminal (2) used by a patient for training for treatment and a supporter terminal (3-n) of a supporter of the patient via a network (N), the server device (4) having a message database (422) for registering messages effective for the treatment of the patient, the treatment support method including:
displaying the messages registered in the message database (422) on the supporter terminal (3-n) via the network (N) in a selectable manner in response to a notification from the patient terminal (2) via the network (N) of a start of the training of the patient; and
displaying a message selected by the supporter with the supporter terminal (3-n) on the patient terminal (2) via the network (N) as a message from the supporter.

A treatment support method according to an eleventh aspect of the present invention is performed in a treatment support system (1) including: a server device (4) connected to a patient terminal (2) used by a patient for training for treatment via a network (N) and having a message database (422) for registering messages effective for the treatment of the patient; and a supporter terminal (3-n) of a supporter of the patient connected to the server device (4) via the network (N),
wherein in response to a notification from the patient terminal (2) via the network (N) of a start of the training of the patient, the server device (4) transmits display data for displaying the messages registered in the message database (422) in a selectable manner to the supporter terminal (3-n) via the network (N),
wherein in response to receiving the display data transmitted from the server device (4) via the network (N), the supporter terminal (3-n) displays the messages effective for the treatment of the patient on a display unit (32) in a selectable manner based on the display data,
wherein the supporter terminal (3-n) transmits a message selection command indicating a message selected by the supporter from among the messages displayed on the display unit (32) to the server device (4) via the network (N), and
wherein in response to receiving the message selection command transmitted from the supporter terminal (3-n) via the network (N), the server device (4) causes the message specified from the message selection command to be displayed on the patient terminal (2) via the network (N) as a message from the supporter.

A program according to a twelfth aspect of the present invention causes a computer to execute
a procedure for displaying messages effective for treatment of a patient on a display unit (32) in a selectable manner, the messages prepared in advance, when being notified that the patient starts training for the treatment using a patient terminal (2) via a network (N); and
a procedure for causing a message selected by a supporter of the patient from among the messages displayed on the display unit (32) to be displayed on the patient terminal (2) via the network (N) as a message from the supporter.

A program according to a thirteenth aspect of the present invention causes a computer of a server device (4), which is connected to a patient terminal (2) used by a patient for training for treatment and a supporter terminal (3-n) of a supporter of the patient via a network (N), and includes a message database (422) for registering messages effective for the treatment of the patient, to execute
a procedure for displaying the messages registered in the message database (422) on the supporter terminal (3-n) in a selectable manner in response to a notification from the patient terminal (2) via the network (N) of a start of the training of the patient; and
a procedure for causing a message selected by a supporter with the supporter terminal (3-n) to be displayed on the patient terminal (2) via the network (N) as a message from the supporter.

### EFFECTS OF THE INVENTION

According to the present invention, it is possible to provide the server device, the treatment support system, the treatment support method, and the program capable of preventing the deterioration of the symptoms of the patient.

### BRIEF DESCRIPTION OF THE DRAWINGS

FIG. 1 is a diagram showing a configuration example of a treatment support system according to a present embodiment.
FIG. 2 is a block diagram showing a configuration example of a patient terminal.
FIG. 3 is a diagram showing a display example of a play screen.
FIG. 4 is a block diagram showing a configuration example of a supporter terminal.
FIG. 5 is a diagram showing a display example of a message selection screen.
FIG. 6 is a block diagram showing a configuration example of a server device.
FIG. 7 is a flowchart showing details of a play start notification process.
FIG. 8 is a flowchart showing details of a first treatment support process.
FIG. 9 is a flowchart showing a continuation of the first treatment support process.
FIG. 10 is a flowchart showing details of a second treatment support process.
FIG. 11 is a flowchart showing details of a first disengagement sign determination process.
FIG. 12 is a flowchart showing a continuation of the first disengagement sign determination process.
FIG. 13 is a flowchart showing the continuation of the first disengagement sign determination process.
FIG. 14 is a flowchart showing details of a mental state determination process.
FIG. 15 is a flowchart showing a continuation of the mental state determination process.
FIG. 16 is a flowchart showing the continuation of the mental state determination process.
FIG. 17 is a flowchart showing a continuation of a balance determination process.
FIG. 18 is a flowchart showing the continuation of the balance determination process.
FIG. 19 is a flowchart showing the continuation of the balance determination process.
FIG. 20 is a flowchart showing details of a treatment activity determination process.
FIG. 21 is a flowchart showing a continuation of the treatment activity determination process.
FIG. 22 is a flowchart showing the continuation of the treatment activity determination process.
FIG. 23 is a flowchart showing details of a second disengagement sign determination process.
FIG. 24 is a flowchart showing a continuation of the second disengagement sign determination process.
FIG. 25 is a flowchart showing the continuation of the second disengagement sign determination process.

### MODE FOR CARRYING OUT THE INVENTION

Hereinafter, the best mode for carrying out the present invention will be described.

First, a configuration of a treatment support system according to an embodiment of the present invention will be described with reference to the drawings.

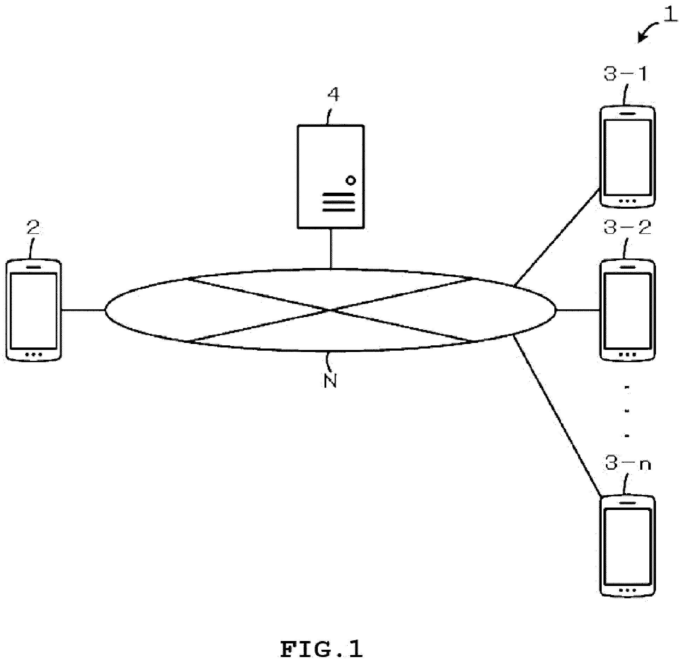

FIG. 1 is a diagram showing a configuration example of a treatment support system according to the present embodiment.

As shown in FIG. 1, the treatment support system 1 includes a patient terminal 2, supporter terminals 3-n (n is a natural number), and a server device 4, which are connected to each other via a network N such as the Internet so as to be able to communicate with each other.

The patient terminal 2 is composed of, for example, a general-purpose smartphone, a tablet computer, a personal computer, and the like. The patient terminal 2 is used, for example, by a patient with Mild Cognitive Impairment (MCI) for training for the treatment.

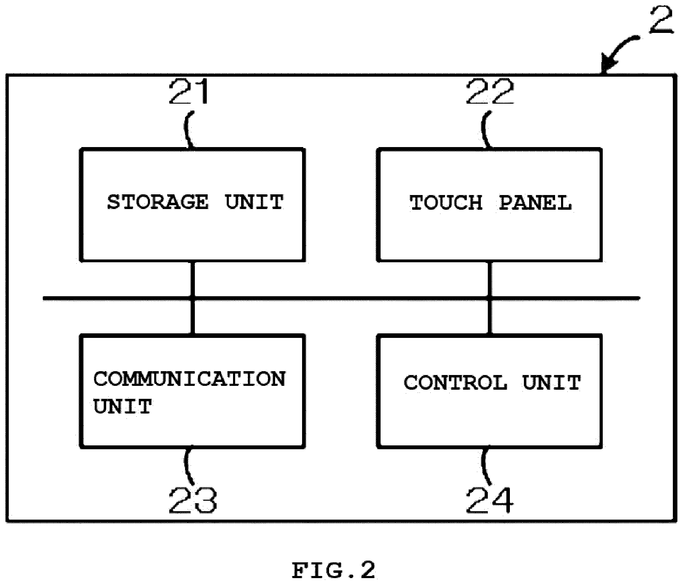

FIG. 2 is a block diagram showing a configuration example of a patient terminal.

As shown in FIG. 2, the patient terminal 2 includes a storage unit 21, a touch panel 22, a communication unit 23, and a control unit 24, which are connected to each other via a bus or the like.

The storage unit 21 is composed of, for example, a non-volatile memory such as a general-purpose flash memory. Various application programs such as an application program for treating the patient with MCI (hereinafter referred to as a "treatment program") are installed in the storage unit 21. The treatment program includes various games and the like for training the patient's brain to prevent development of MCI.

The storage unit 21 stores a usage log such as the usage time and the number of times of activation of the treatment program, content of answer to various questionnaires and the like. The content of answer to the various questionnaires and the like include the number of times of answer and content of answer to a questionnaire about a lifestyle habit, content of answer to a questionnaire about a treatment activity, and content of answer to a questionnaire about a patient's mood (hereinafter referred to as a "mental log"). The mental log includes the patient's mood on a daily basis, a scale value of 0 to 100 representing the degree of mood, and the like.

The touch panel 22 is composed of, for example, a general-purpose touch panel in which a liquid crystal display device and a pointing device are combined. The touch panel 22 displays various screens and receives various operations by the patient. In the present embodiment, the touch panel 22 displays a treatment menu by the treatment program. The patient plays various games and the like included in the treatment program on a play screen displayed on the touch panel 22 in accordance with the treatment menu.

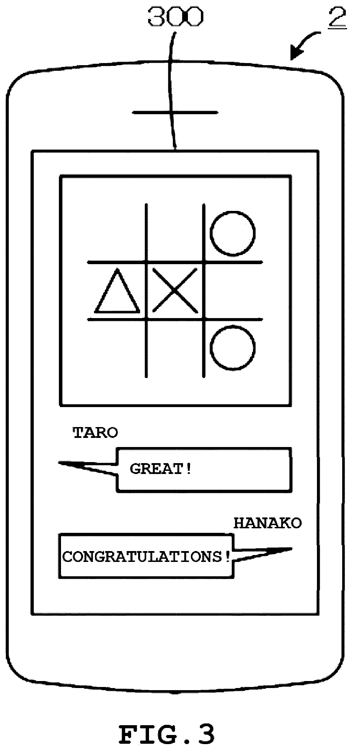

FIG. 3 is a diagram showing a display example of a play screen.

As shown in FIG. 3, a game being played by the patient is displayed on a play screen 300. Further, each of display names of neighboring supporters who are closely related to the patient, such as a patient's families or relatives, and each of messages (feedback messages) from the supporters who support the patient playing the game, such as "Great!" and "Congratulations!", are displayed at the bottom of the play screen 300.

In addition, the patient inputs an answer to each of the various questionnaires included in the treatment program on the touch panel 22. For example, the patient inputs items related to "meal", "sleep", "activity", and "medication" in the questionnaire about the lifestyle habit. Specifically, the patient inputs the amount of carbohydrate intake per day, the presence or absence of protein intake, the presence or absence of intake of supplementary food, and the like with respect to "meal". Further, the patient inputs the sleep time and the degree of deep sleep with respect to "sleep". Furthermore, the patient inputs the amount of activity (number of steps) per day with respect to "activity". Moreover, the patient inputs the presence or absence of medication with respect to "medication". In addition, in the questionnaire about the treatment activity, the patient inputs impressions and motivations regarding the training and efforts to improve the lifestyle habit such as "dissatisfied", "bored", and "reduced motivation". In addition, the patient inputs the mental log consisting of moods such as "anxious", "irritated", "depressed", "sad", and the like, and the scale value thereof in the questionnaire about the patient's mood. Hereinafter, the mental log input by the patient from a viewpoint of the patient is referred to as a "patient viewpoint mental log".

The communication unit 23 shown in FIG. 2 is composed of, for example, a general-purpose wireless communication device. The communication unit 23 transmits, to the server device 4 via the network N, a first play start notification notifying the server device 4 that the patient has started playing the game. The communication unit 23 receives the first to sixth message display commands transmitted from the server device 4 via the network N.

The communication unit 23 transmits, to the server device 4 via the network N, the usage log notification notifying the usage log or the like of the treatment program, and an answer content notification notifying the content of answer to the questionnaire about the treatment activity.

The control unit 24 is composed of, for example, a CPU (Central Processing Unit), a ROM (Read Only Memory), and a RAM (Random Access Memory). The CPU uses the RAM as a work memory, and controls various operations of the patient terminal 2 by appropriately executing various programs and the like stored in the ROM and the storage unit 21.

In the present embodiment, the control unit 24 activates the treatment program stored in the storage unit 21 in response to the patient tapping an icon indicating the treatment program displayed on the touch panel 22, and displays a top screen of the treatment program on the touch panel 22. Next, the control unit 24 causes the play screen 300 for playing a game to be displayed on the touch panel 22 in response to the patient selecting the game to be play on the top screen. Further, the control unit 24 transmits, from the communication unit 23 to the server device 4 via the network N, the first play start notification including the patient identifier capable of identifying the patient who has started the game.

In response to receiving the first message display command transmitted from the server device 4 via the network N with the communication unit 23, the control unit 24 causes the display name of the supporter specified by the message display command and the message selected by the supporter to be displayed on the play screen 300 below the game being played by the patient.

Each time executing the treatment program, the control unit 24 stores the usage time and the number of times of activation in the storage unit 21. In addition, the control unit 24 performs the questionnaire about the lifestyle habit and the questionnaire about the patient's mood every day in the treatment program. Then, the control unit 24 stores, in the storage unit 21, the number of times of answer and the content of answer to the questionnaire about the lifestyle habit, and the content of the answer to the questionnaire about the patient's mood (patient viewpoint mental log) which are input by the patient on the touch panel 22. Furthermore, in the treatment program, the control unit 24 performs the questionnaire about the treatment activity to the patient once a week.

In response to a predetermined transmission timing (for example, 12:00 PM) of the day, the control unit 24 reads, from the storage unit 21, the usage log such as the usage time and the number of times of activation of the treatment program on the day, the number of times of answer and the content of answer to the questionnaire about the lifestyle habit, the patient viewpoint mental log, and the like. Next, the control unit 24 obtains an implementation status of the treatment activity from the number of times of activation of the treatment program, and the number of times and the content of answer to the questionnaire about the lifestyle habit. Then, the control unit 24 transmits, from the communication unit 23 to the server device 4 via the network N, the usage log notification (usage history of the patient terminal 2) including the implementation status of the treatment activity, the usage log such as the usage time and the number of times of activation of the treatment program, the date, and the patient identifier.

Further, in response to the patient inputting the answer content to the questionnaire about the treatment activity on the touch panel 22, the control unit 24 transmits the answer content notification including the patient identifier, the answer content to the questionnaire about the treatment activity, and the like from the communication unit 23 to the server device 4 via the network N.

In response to receiving each of the second to sixth message display commands transmitted from the server device 4 via the network N with the communication unit 23, the control unit 24 causes the display name of the supporter identified from each of the second to sixth message display commands and the message selected by the supporter to be displayed on the touch panel 22.

Each of the supporter terminals 3-n shown in FIG. 1 is composed of a general-purpose smart phone, a tablet computer, a personal computer, and the like, similarly to the patient terminal 2. The supporter terminal 3-n is possessed by each of the neighboring supporters who are closely related to the patient, such as a patient's families or relatives.

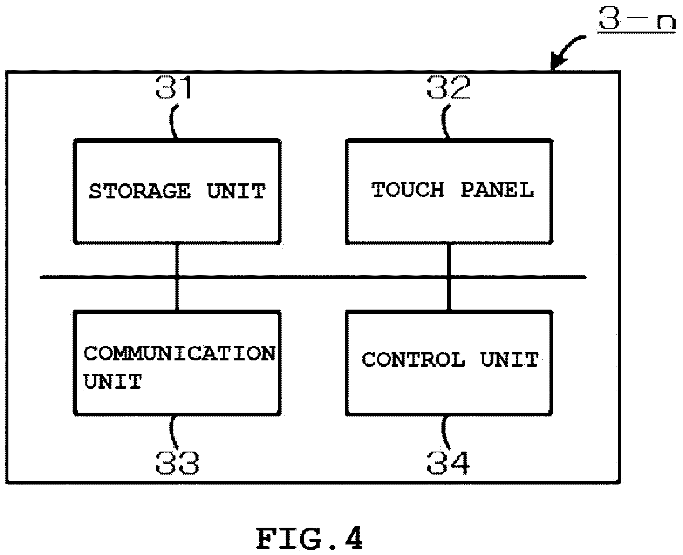

FIG. 4 is a block diagram showing a configuration example of a supporter terminal.

As shown in FIG. 4, the supporter terminal 3-n includes a storage unit 31, a touch panel 32, a communication unit 33, and a control unit (terminal control unit) 34, which are connected to each other via a bus or the like.

Similarly to the storage unit 21, the storage unit 31 is composed of a non-volatile memory such as a general-purpose flash memory. Various application programs such as an application program for supporting the patient (hereinafter, referred to as "support program") are installed in the storage unit 31.

Similarly to the touch panel 22, the touch panel 32 is composed of a general-purpose touch panel or the like. The touch panel 32 displays various screens and receives various operations by the supporter. In the present embodiment, a first message selection screen for selecting a message for supporting the patient playing the game from among a plurality of types thereof is displayed on the touch panel 32.

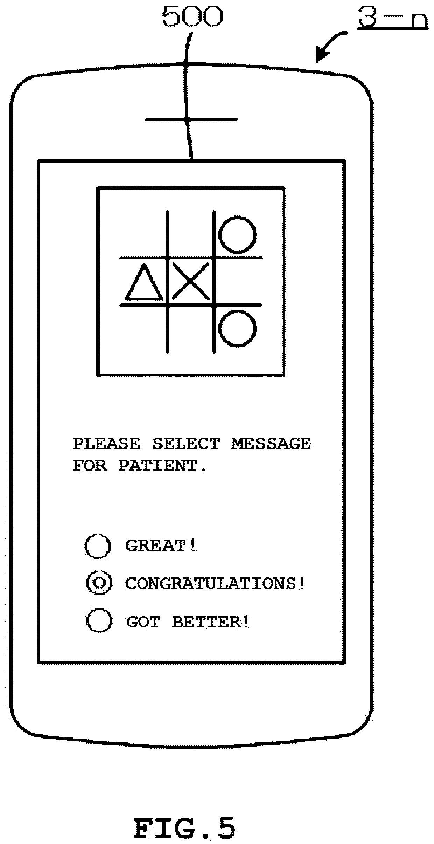

FIG. 5 is a diagram showing a display example of a message selection screen.

As shown in FIG. 5, messages such as "Great!", "Congratulations!", and "Good!" are displayed on the first message selection screen 500 in a selectable manner, and a situation of the game being played by the patient is displayed above the messages. Thus, the situation of the game being played by the patient is also shared with the supporter. The supporter taps and selects the message for supporting the patient playing the game on the first message selection screen 500, considering the situation of the game being played by the patient.

The touch panel 32 also displays a second message selection screen for selecting, from among a plurality of types of messages, a message for supporting the patient who is recognized as having a deteriorated mental state or an indication of departure from the training (hereinafter, referred to as "disengagement sign"). In the second message selection screen, the supporter taps and selects the message for supporting the patient who is recognized as having the disengagement sign.

Further, the touch panel 32 displays a third message selection screen for selecting, from among a plurality of types of messages, a message for encouraging or comforting the patient who is recognized as having the deteriorated mental state. In the third message selection screen, the supporter taps and selects the message for encouraging or comforting the patient who is recognized as having the deteriorated mental state.

Further, the touch panel 32 displays a fourth message selection screen for selecting, from among a plurality of types of messages, a message for supporting or encouraging the patient who is recognized as having a deteriorated balance of the treatment activity, such as "Work hard!". In the fourth message selection screen, the supporter taps and selects the message for supporting or encouraging the patient who is recognized as having the deteriorated balance of the treatment activity.

Furthermore, the touch panel 32 displays a fifth message selection screen for selecting, from among a plurality of types of messages, a message for supporting the patient who is trying to do his/her best in the treatment activity, such as "Work hard!". In the fifth message selection screen, the supporter taps and selects the message for supporting the patient who is trying to do his/her best in the treatment activity.

The touch panel 32 also displays a sixth message selection screen for selecting, from among a plurality of types of messages, a message for praising the patient who is sufficiently performing the treatment activity, such as "great!" or "Like!". In the sixth message selection screen, the supporter taps and selects the message praising the patient who is sufficiently performing the treatment activity.

Further, the supporter inputs an answer to each of the various questionnaires included in the support program on the touch panel 32. For example, the supporter inputs the mental log consisting of patient's moods such as "anxious", "irritated", "depressed", and "sad", and the scale value thereof in the questionnaire about the patient's mood. Hereinafter, the mental log input by the supporter from a viewpoint of the supporter is referred to as "supporter viewpoint mental log".

Similarly to the communication unit 23, the communication unit 33 shown in FIG. 2 is composed of a general-purpose wireless communication device or the like. The communication unit 33 receives a second play start notification transmitted from the server device 4 via the network N, the display data for displaying the first message selection screen 500, the display data for displaying the second message selection screen, and the like. In addition, the communication unit 33 transmits a support participation request which is a request of the supporter to participate in the support for the patient playing the game, the message selection command indicating a message selected by the supporter, and a supporter viewpoint mental log notification notifying the supporter viewpoint mental log to the server device 4 via the network N.

Similarly to the control unit 24, the control unit 34 is composed of a CPU, a ROM, a RAM, and the like. The CPU controls the various operations of the supporter terminal 3-n by appropriately executing the various programs and the like stored in the ROM and the storage unit 31 using the RAM as a work memory.

In the present embodiment, in response to receiving the second play start notification transmitted from the server device 4 via the network N with the communication unit 33, the control unit 34 causes the fact that the patient has started playing the game to be displayed on the touch panel 32, causes a sound from a speaker (not shown) to be outputted, or causes a push notification to be notified to the supporter using a vibrator (not shown).

Next, in response to the supporter tapping the icon indicating the support program displayed on the touch panel 32 and indicating to participate in the support for the patient playing the game, the control unit 34 starts the support program stored in the storage unit 31 and transmits the support participation request including a supporter identifier capable of identifying the supporter from the communication unit 33 to the server device 4 via the network N.

In response to receiving the display data of the first message selection screen 500 transmitted from the server device 4 via the network N with the communication unit 32, the control unit 34 causes the first message selection screen 500 to be displayed on the touch panel 32 based on the display data. Then, in response to the supporter tapping and selecting a message on the first message selection screen 500, the control unit 34 transmits a first message selection command including the message selected by the supporter and the supporter identifier capable of identifying the supporter from the communication unit 32 via the network N.

In addition, the control unit 34 performs the questionnaire about the patient's mood to the supporter every day in the support program. The control unit 34 transmits the supporter viewpoint mental log notification including the supporter viewpoint mental log and the supporter identifier from the communication unit 33 to the server device 4 via the network N in response to the supporter inputting the content of answer (patient viewpoint mental log) to the questionnaire about the patient's mood on the touch panel 32.

Further, in response to receiving the display data of each of the second to sixth message selection screens transmitted from the server device 4 via the network N with the communication unit 32, the control unit 34 causes each of the second to sixth message selection screens to be displayed on the touch panel 32 based on the display data. Then, in response to the supporter tapping and selecting the message on each of the second to sixth message selection screens, the control unit 34 transmits each of second to sixth message selection commands, each of which includes the message selected by the supporter and the supporter identifier capable of identifying the supporter, from the communication unit 32 via the network N.

The server device 4 shown in FIG. 1 is composed of, for example, a general-purpose server computer, a general-purpose data base (DataBase; DB), and the like.

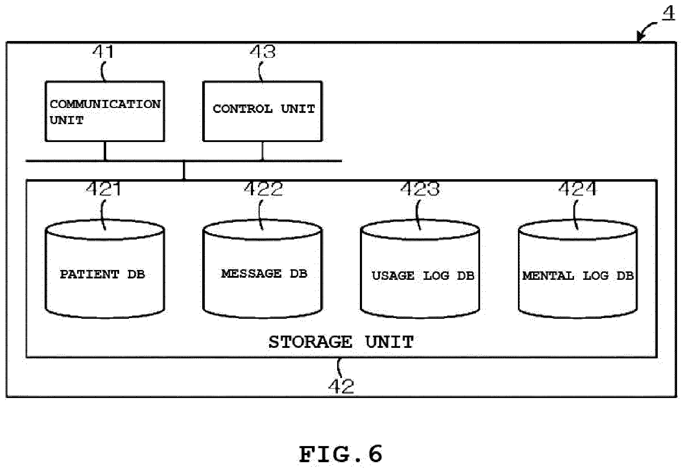

FIG. 6 is a block diagram showing a configuration example of a server device.

As shown in FIG. 6, the server device 4 includes a communication unit 41, a storage unit 42, and a control unit (server control unit) 43, which are connected to each other via a bus or the like.

The communication unit 41 is composed of, for example, a NIC (Network Interface Card) or the like. The communication unit 41 receives the first play start notification, the usage log notification, and the answer content notification transmitted from the patient terminal 2 via the network N. The communication unit 41 transmits, to the supporter terminal 3-n via the network N, the second play start notification notifying the supporter terminal 3-n that the patient has started playing the game and the display data such as the first to sixth message selection screens 500. Further, the communication unit 41 receives the support participation request, the first to sixth message selection commands, and the supporter viewpoint mental log notification which are transmitted from the supporter terminal 3-n via the network N. The communication unit 41 transmits the first to sixth message display commands for instructing the display of the message selected by the supporter to the patient terminal 2 via the network N.

The storage unit 42 is composed of, for example, a general-purpose hard disk drive. In the present embodiment, the storage unit 42 includes a patient DB 421 and a message DB 422.

Further, a usage log DB 423 is provided in the storage unit 42 for each patient, and a mental log DB 424 is provided in the storage unit 42 for each supporter.

The patient DB 421 registers, for each patient, information on the patient (hereinafter referred to as "patient information"), information capable of identifying the supporter terminal 3-n of the neighboring supporter of the patient (hereinafter referred to as "supporter information"), and a treatment menu in association with each other. The patient information includes, for example, the patient identifier capable of identifying the patient, a name and characteristics (for example, gender, age, medical condition, personality, and the like) of the patient. The supporter information includes, for example, the supporter identifier capable of identifying the supporter, the display name of the supporter, a relationship with the patient, an e-mail address, and the like.

The message DB 422 registers a plurality of types of messages from the supporter, who supports, encourages, comforts, and praises the patient, in association with the characteristics of the patient.

In order to improve the symptoms of the patient with MCI, it is essential to provide the support from each of the neighboring supporters who are closely related to the patient, such as the patient's families and relatives. The messages from the supporter who supports, encourages, comforts, and praises the patient are also very helpful in improving the symptoms of the patient with MCI because the messages can give the patient confidence and maintain motivation of the patient to train. However, the patient with MCI has symptoms such as memory impairment and disorientation, and unstable emotional expressions such as depression and agitation. For this reason, the patient may become emotional or deeply hurt by mistaking the meaning of the message from the supporter, thereby losing the motivation of the patient for the training and, on the contrary, worsening the symptoms of the patient with MCI. Therefore, in the present embodiment, only the messages effective for the treatment of the patient with MCI are prepared in advance in the message DB 422, such that the supporter selects a message for supporting, encouraging, comforting, or praising the patient from the message DB 422 to interact with the patient. For this reason, the supporter can prevent a message that may deteriorate the symptoms of the patient with MCI from being generated.

The messages registered in the message DB 422 are supervised and updated as appropriate by experts in a group therapy, such as medical specialists and therapists, who have relevant knowledge of the treatment of the patient with MCI.

The usage log DB 423 correspondingly registers the usage time, the number of times of activation and the frequency of use of the treatment program, the implementation status of the treatment activity, the patient viewpoint mental log, the degree of achievement of the treatment menu, the degree of achievement of the lifestyle habit, and the like for each date.

The mental log DB 424 correspondingly registers the supporter viewpoint mental log and the like for each date.

The control unit 43 is composed of, for example, a CPU, a ROM, a RAM, and the like. The CPU controls the various operations of the server device 4 by appropriately executing the various programs and the like stored in the ROM and the storage unit 42 using the RAM as a work memory. The RAM is provided with a disengagement sign flag and the like indicating that the disengagement sign is recognized in the patient.

In the present embodiment, in response to receiving the first play start notification transmitted from the patient terminal 2 via the network N with the communication unit 41, the control unit 43 reads (reads out) the patient information and the supporter information corresponding to the patient identifier included in the first play start notification from the patient DB 421. Next, the control unit 43 transmits the second play start notification from the communication unit 41 via the network N to the supporter terminal 3-n possessed by the supporter identified from the supporter information.

In response to receiving the support participation request transmitted from the supporter terminal 3-n via the network N with the communication unit 41, the control unit 43 detects, from the message DB 422, the plurality of types of messages corresponding to the characteristics of the patient included in the patient information read from the patient DB 421. Subsequently, the control unit 43 generates, from the plurality of types of messages detected from the message DB 422, the display data of the first message selection screen 500 for selecting the message for supporting the patient playing the game. Then, the control unit 43 transmits the display data of the first message selection screen 500 from the communication unit 41 via the network N to the supporter terminal 3-n possessed by the supporter identified from the supporter identifier included in the support participation request.

In response to receiving the first message selection command transmitted from the supporter terminal 3-n via the network N with the communication unit 41, the control unit 43 transmits the first message display command including the display name of the supporter identified from the supporter information corresponding to the supporter identifier included in the first message selection command and the message included in the first message selection command from the communication unit 41 to the patient terminal 2 via the network N.

In response to receiving the usage log notification transmitted from the patient terminal 2 via the network N with the communication unit 41, the control unit 43 detects, from the storage unit 42, the usage log DB 423 of the patient identified from the patient identifier included in the usage log notification. Subsequently, the control unit 43 correspondingly registers the date, the usage log such as the usage time and the number of times of activation of the treatment program, the implementation status of the treatment activity, the mental log, and the like included in the usage log notification in the usage log DB 423 detected from the storage unit 42.

In response to receiving the supporter viewpoint mental log notification transmitted from the supporter terminal 3-n via the network N, the control unit 43 detects, from the storage unit 42, the mental log DB 424 of the supporter identified from the supporter identifier included in the supporter viewpoint mental log notification. Subsequently, the control unit 43 registers the supporter viewpoint mental log included in the supporter viewpoint mental log notification in the mental log DB 424 detected from the storage unit 42.

Then, the control unit 43 executes various processes such as the first disengagement sign determination process, the mental state determination process, the balance determination process, and the treatment activity determination process based on the usage log such as the usage time and the number of times of activation of the treatment program, the implementation status of the treatment activity, the patient viewpoint mental log registered in the usage log DB 423, the supporter viewpoint mental log registered in the mental log DB 424, and the like.

### [First disengagement sign determination process]

In the first disengagement sign determination process, first, the control unit 43 determines the frequency of use on the day from the usage log such as the usage time and the number of times of activation of the treatment program corresponding to the latest date. Specifically, the control unit 43 determines that the frequency of use is "non-use" when the number of times of activation of the treatment program is 0 or the usage time is less than 5 minutes. Further, the control unit 43 determines that the frequency of use is "insufficient" when the usage time is 5 minutes or more and less than 30 minutes. Furthermore, the control unit 43 determines that the frequency of use is "use" when the usage time is 30 minutes or more. Then, the control unit 43 registers the frequency of use of the treatment program in the usage log DB 423 in association with the latest date.

Next, the control unit 43 determines the frequency of use in the latest three days (hereinafter, referred to as "three-days frequency of use"). Specifically, the control unit 43 reads the frequency of use of the treatment program for the latest three days from the usage log DB 423. Subsequently, the control unit 43 specifies the number of days in which the frequency of use of the treatment program is "use" in the latest three days. If the number of days in which the frequency of use of the treatment program is "use" is 0 days, the control unit 43 determines that the three-days frequency of use is "non-use". Further, if the number of days in which the frequency of use of the treatment program is "use" is 1 day to 2 days, the control unit 43 determines that the three-days frequency of use is "insufficient". Furthermore, if the number of days in which the frequency of use of the treatment program is "use" is 3 days, the control unit 43 determines that the three-days frequency of use is "use".

Further, the control unit 43 determines the frequency of use in the latest week (hereinafter, referred to as "the one-week frequency of use"). Specifically, the control unit 43 reads the frequency of use of the treatment program for the latest week from the usage log DB 423. Subsequently, the control unit 43 specifies the number of days in which the frequency of use of the treatment program is "use" in the latest week. If the number of days in which the frequency of use of the treatment program is "use" is 0 days, the control unit 43 determines that the one-week frequency of use is "non-use". Further, if the number of days in which the frequency of use of the treatment program is "use" is 1 day to 3 days, the control unit 43 determines that the one-week frequency of use is "insufficient". Furthermore, if the number of days in which the frequency of use of the treatment program is "use" is 4 days to 6 days, the control unit 43 determines that the one-week frequency of use is "moderate". Moreover, if the number of days in which the frequency of use of the treatment program is "use" is 7 days, the control unit 43 determines that the one-week frequency of use is "use".

Then, the control unit 43 determines whether or not a sign of disengagement from the training (hereinafter, referred to as "disengagement sign") is recognized in the patient from the three-days frequency of use and the one-week frequency of use. In a case where the three-days frequency of use is "non-use" or "insufficient", the control unit 43 assumes that a bad state continues if the one-week frequency of use is "non-use" or "insufficient". Further, in the case, the control unit 43 assumes that the state is deteriorated even if the one-week frequency of use is "moderate". As a result, the control unit 43 determines that the disengagement sign is recognized in the patient and turns on the disengagement sign flag.

On the other hand, in a case where the three-days frequency of use is "use", the control unit 43 assumes that a good state continues if the one-week frequency of use is "moderate" or "use". Further, in the case, the control unit 43 assumes that the state is improved even if the one-week frequency of use is "non-use" or "insufficient". As a result, the control unit 43 determines that the disengagement sign is not recognized in the patient from the frequency of use of the treatment program, and reads the treatment menu corresponding to the patient identifier included in the usage log from the patient DB 421.

Then, the control unit 43 compares the implementation status of the treatment activity corresponding to the latest date with the treatment menu read from the patient DB 421, and determines the degree of achievement by obtaining an achievement rate of the treatment menu. Specifically, the control unit 43 determines that the degree of achievement is "not achieved" if the achievement rate of the treatment menus is 0% or more and less than 25%. Further, the control unit 43 determines that the degree of achievement is "insufficient" if the achievement rate is 25% or more and less than 50%. Furthermore, the control unit 43 determines that the degree of achievement is "minimum" if the achievement rate is 50% or more and less than 75%. Moreover, the control unit 43 determines that the degree of achievement is "moderate" if the achievement rate is 75% or more and less than 100%. In addition, the control unit 43 determines that the degree of achievement is "achievement" if the achievement rate is 100% or more. Then, the control unit 43 registers the degree of achievement of the treatment menu in the usage log DB 423 in association with the latest date.

Next, the control unit 43 determines the degree of achievement in the latest three days (hereinafter, referred to as "three-days treatment menu achievement degree"). Specifically, the control unit 43 reads the degree of achievement of the treatment menu for the latest three days from the usage log DB 423. Subsequently, the control unit 43 specifies the number of days in which the degree of achievement of the treatment menu is "minimum", "moderate", or "achievement" (hereinafter, referred to as "minimum or more") in the latest three days. The control unit 43 determines that the three-days treatment menu achievement degree is "not achieved" if the number of days in which the degree of achievement of the treatment menu is "minimum or more" is 0 days. Further, the control unit 43 determines that the three-days treatment menu achievement degree is "insufficient" if the number of days in which the degree of achievement of the treatment menu is "minimum or more" is 1 day or 2 days. Furthermore, the control unit 43 determines that the three-days treatment menu achievement degree is "minimum" if the number of days in which the degree of achievement of the treatment menu is "minimum or more" is 3 days.

Further, the control unit 43 determines the degree of achievement in the latest week (hereinafter, referred to as "one-week treatment menu achievement degree"). Specifically, the control unit 43 reads the degree of achievement of the treatment menu for the latest week from the usage log DB 423. Subsequently, the control unit 43 specifies the number of days in which the degree of achievement of the treatment menu is "achievement" in the latest week. The control unit 43 determines that the one-week treatment menu achievement degree is "not achieved" if the number of days in which the degree of achievement of the treatment menu is "achievement" is 0 days. Further, the control unit 43 determines that the one-week treatment menu achievement degree is "insufficient" if the number of days in which the degree of achievement of the treatment menu is "achievement" is 1 day to 5 days. Furthermore, the control unit 43 determines that the one-week treatment menu achievement degree is "minimum" if the number of days in which the degree of achievement of the treatment menu is "achievement" is 6 days to 7 days.

Then, the control unit 43 determines whether or not the disengagement sign is recognized in the patient from the three-days treatment menu achievement degree and the one-week treatment menu achievement degree. In a case where the three-days treatment menu achievement degree is "not achieved" or "insufficient", the control unit 43 assumes that a bad state continues if the one-week treatment menu achievement degree is also "not achieved" or "insufficient". Further, in the case, the control unit 43 assumes that the state is deteriorated even if the one-week treatment menu achievement degree is "minimum". As a result, the control unit 43 determines that the disengagement sign is recognized in the patient and turns on the disengagement sign flag.

On the other hand, in a case where the three-days treatment menu achievement degree is "minimum", the control unit 43 assumes that a good state continues if the one-week treatment menu achievement degree is "minimum". Further, in the case, the control unit 43 assumes that the state is improved even if the one-week treatment menu achievement degree is "insufficient". As a result, the control unit 43 determines that the disengagement sign is not recognized in the patient based on not only the frequency of use of the treatment program but also the achievement degree of the treatment menu, and ends the first disengagement sign determination process as it is.

When the control unit 43 determines that the disengagement sign is recognized in the patient, the control unit 43 reads the patient information and the supporter information corresponding to the patient identifier included in the usage log from the patient DB 421. The control unit 43 detects, from the message DB 422, the plurality of types of messages corresponding to the characteristics of the patient included in the patient information read from the patient DB 421. Subsequently, the control unit 43 generates, from the plurality of types of messages detected from the message DB 422, the display data of the second message selection screen for selecting the message for encouraging or comforting the patient who is recognized as having the disengagement sign. Then, the control unit 43 transmits the display data of the second message selection screen from the communication unit 41 via the network N to the supporter terminal 3-n possessed by the supporter identified from the supporter identifier included in the supporter information.

In response to receiving the second message selection command transmitted from the supporter terminal 3-n via the network N with the communication unit 41, the control unit 43 transmits the second message display command including the display name of the supporter identified from the supporter information corresponding to the supporter identifier included in the second message selection command and the message included in the second message selection command from the communication unit 41 to the patient terminal 2 via the network N.

### [Mental state determination process]

In the mental state determination process, first, the control unit 43 determines the mental state from the viewpoint of the patient in the latest three days (hereinafter, referred to as "three-days patient viewpoint mental state"). Specifically, the control unit 43 reads the patient viewpoint mental log for the latest three days from the usage log DB 423. Subsequently, in the latest three days, the control unit 43 specifies the number of days in which the patient's mood has negative emotions such as "anxious", "irritated", "depressed", and "sad". The control unit 43 determines that the three-days patient viewpoint mental state is "no problem" if the number of days in which the patient's mood has the negative emotions is 0 days. Further, the control unit 43 determines that the three-days patient viewpoint mental state is "observation is required" if the number of days in which the patient's mood has the negative emotions is 1 day or 2 days. Furthermore, the control unit 43 determines that the three-days patient viewpoint mental state is "problem exists" if the number of days in which the patient's mood has the negative emotions is 3 days.

Next, the control unit 43 determines the mental state from the viewpoint of the patient in the latest week (hereinafter, referred to as "one-week patient viewpoint mental state"). Specifically, the control unit 43 reads the patient viewpoint mental log for the latest week from the usage log DB 423. Subsequently, the control unit 43 specifies the number of days in which the patient's mood has the negative emotions such as "anxious", "irritated", "depressed", and "sad" in the latest week. The control unit 43 determines that the one-week patient viewpoint mental state is "no problem" if the number of days in which the patient's mood has the negative emotions is 0 days. Further, the control unit 43 determines that the one-week patient viewpoint mental state is "observation is required" if the number of days in which the patient's mood has the negative emotions is 1 day to 3 days. Furthermore, the control unit 43 determines that the one-week patient viewpoint mental state is "problem exists" if the number of days in which the patient's mood has the negative emotions is 4 days to 7 days.

Then, the control unit 43 determines, from the three-days patient viewpoint mental state and the one-week patient viewpoint mental state, whether or not the deterioration of the mental state is recognized in the patient from the viewpoint of the patient. In a case where the three-days patient viewpoint mental state is "problem exists", the control unit 43 assumes that a bad state continues if the one-week patient viewpoint mental state is also "problem exists". Further, in the case, the control unit 43 assumes that the state is deteriorated even if the one-week patient viewpoint mental state is "observation is required". As a result, the control unit 43 determines that the deterioration of the mental state is recognized in the patient from the viewpoint of the patient.

On the other hand, in a case where the three-days patient viewpoint mental state is "no problem" or "observation is required", the control unit 43 assumes that a good state continues if the one-week patient viewpoint mental state is "no problem" or "observation is required". Further, in the case, the control unit 43 assumes that the state is improved even if the one-week patient viewpoint mental state is "problem exists". As a result, the control unit 43 determines that the deterioration of the mental state is not recognized in the patient from the viewpoint of the patient.

When the control unit 43 determines that the deterioration of the mental state is not recognized in the patient from the viewpoint of the patient, the control unit 43 detects, from the storage unit 42, the mental log DB 424 of the supporter identified from the supporter identifier included in the supporter viewpoint mental log.

The control unit 43 determines the mental state from the viewpoint of the supporter in the latest three days (hereinafter, referred to as "three-days supporter viewpoint mental state"). Specifically, the control unit 43 reads the supporter viewpoint mental log for the latest three days from the mental log DB 424. Subsequently, in the latest three days, the control unit 43 specifies the number of days in which the patient's mood has the negative emotions such as "anxious", "irritated", "depressed", and "sad". The control unit 43 determines that the three-days supporter viewpoint mental state is "no problem" if the number of days in which the patient's mood has the negative emotions is 0 days. Further, the control unit 43 determines that the three-days supporter viewpoint mental state is "observation is required" if the number of days in which the patient's mood has the negative emotions is 1 day or 2 days. Furthermore, the control unit 43 determines that the three-days supporter viewpoint mental state is "problem exists" if the number of days in which the patient's mood has the negative emotions is 3 days.

Next, the control unit 43 determines the mental state from the viewpoint of the supporter in the latest week (hereinafter, referred to as "one-week supporter viewpoint mental state"). Specifically, the control unit 43 reads the supporter viewpoint mental log for the latest week from the usage log DB 423. Subsequently, the control unit 43 specifies the number of days in which the patient's mood has the negative emotions such as "anxious", "irritated", "depressed", and "sad" in the latest week. The control unit 43 determines that the one-week supporter viewpoint mental state is "no problem" if the number of days in which the patient's mood has the negative emotions is 0 days. Further, the control unit 43 determines that the one-week supporter viewpoint mental state is "observation is required" if the number of days in which the patient's mood has the negative emotions is 1 day to 3 days. Furthermore, the control unit 43 determines that the one-week supporter viewpoint mental state is "problem exists" if the number of days in which the patient's mood has the negative emotions is 4 days to 7 days.

Then, the control unit 43 determines, from the three-days supporter viewpoint mental state and the one-week supporter viewpoint mental state, whether or not the deterioration of the mental state is recognized in the patient from the viewpoint of the supporter. Specifically, in a case where the three-days supporter viewpoint mental state is "problem exists", the control unit 43 assumes that a bad state continues if the one-week supporter viewpoint mental state is also "problem exists". Further, in the case, the control unit 43 assumes that the state is deteriorated even if the one-week supporter viewpoint mental state is "observation is required". As a result, the control unit 43 determines that the deterioration of the mental state is recognized in the patient from the viewpoint of the supporter.

On the other hand, in a case where the three-days supporter viewpoint mental state is "no problem" or "observation is required", the control unit 43 assumes that a good state continues if the one-week supporter viewpoint mental state is "no problem" or "observation is required". Further, in the case, the control unit 43 assumes that the state is improved even if the one-week supporter viewpoint mental state is "problem exists". As a result, when the control unit 43 determines that the deterioration of the mental state is not recognized in the patient not only from the viewpoint of the patient but also from the viewpoint of the supporter, the control unit 43 ends the mental state determination process as it is.

When the control unit 43 determines that the mental state of the patient is deteriorated from either the patient viewpoint or the supporter viewpoint, the control unit 43 reads the patient information and the supporter information corresponding to the patient identifier included in the usage log from the patient DB 421. The control unit 43 detects, from the message DB 422, the plurality of types of messages corresponding to the characteristics of the patient included in the patient information read from the patient DB 421. Subsequently, the control unit 43 generates, from the plurality of types of messages detected from the message DB 422, the display data of the third message selection screen for selecting the message for encouraging or comforting the patient who is recognized to be deteriorated in mental state. Then, the control unit 43 transmits the display data of the third message selection screen from the communication unit 41 via the network N to the supporter terminal 3-n possessed by the supporter identified from the supporter identifier included in the supporter information.

In response to receiving the third message selection command transmitted from the supporter terminal 3-n via the network N with the communication unit 41, the control unit 43 transmits the third message display command including the display name of the supporter identified from the supporter information corresponding to the supporter identifier included in the third message selection command and the message included in the third message selection command from the communication unit 41 to the patient terminal 2 via the network N.

### [Balance determination process]

In the balance determination process, first, the control unit 43 determines whether or not the disengagement sign flag is on. When the disengagement sign flag is on, the control unit 43 assumes that the disengagement sign is recognized in the patient, and ends the balance determination process as it is.

On the other hand, when the disengagement sign flag is off, that is, when no disengagement sign is recognized in the patient, the control unit 43 determines the degree of achievement of the lifestyle habit by obtaining the achievement rate of the lifestyle habit of the patient from the number of times of answer to the questionnaire about the lifestyle habit and the content of the answer included in the implementation status of the treatment activity corresponding to the latest date. Specifically, the control unit 43 determines the degree of achievement by obtaining the achievement rate of the lifestyle habit based on the number of times of answer to the questionnaire about the lifestyle habit, whether or not the amount of carbohydrate intake per day is equal to or less than 150g, whether or not the protein is ingested, whether or not the supplementary food is ingested, whether or not the sleep time is equal to or more than 7 hours, whether or not the sleep is deep based on the degree of deep sleep, whether or not the activity per day (the number of steps) is equal to or more than 5000 steps, whether or not the medication is taken, and the like. The control unit 43 determines that the degree of achievement of the lifestyle habit is "not achieved" if the achievement rate of the lifestyle habit is 0% or more and less than 25%. Further, the control unit 43 determines that the degree of achievement of the lifestyle habit is "insufficient" if the achievement rate is 25% or more and less than 50%. Furthermore, the control unit 43 determines that the degree of achievement of the lifestyle habit is "minimum" if the achievement rate is 50% or more and less than 75%. Moreover, the control unit 43 determines that the degree of achievement of the lifestyle habit is "moderate" if the achievement rate is 75% or more and less than 100%. In addition, the control unit 43 determines that the degree of achievement of the lifestyle habit is "achievement" if the achievement rate is 100% or more.

Then, the control unit 43 registers the degree of achievement of the lifestyle habit in the usage log DB 423 in association with the latest date.

Next, the control unit 43 determines the degree of achievement in the latest three days (hereinafter, referred to as "three-days lifestyle habit achievement degree"). Specifically, the control unit 43 reads the degree of achievement of the lifestyle habit for the latest three days from the usage log DB 423. Subsequently, the control unit 43 specifies the number of days in which the degree of achievement of the lifestyle habit is "minimum", "moderate", or "achievement" (hereinafter referred to as "minimum or more") in the latest three days. The control unit 43 determines that the three-days lifestyle habit achievement degree is "not achieved" if the number of days in which the degree of achievement of the lifestyle habit is "minimum or more" is 0 days. Further, the control unit 43 determines that the three-days lifestyle habit achievement degree is "insufficient" if the number of days in which the degree of achievement of the lifestyle habit is "minimum or more" is 1 day or 2 days. Furthermore, the control unit 43 determines that the three-days lifestyle habit achievement degree is "minimum" if the number of days in which the degree of achievement of the lifestyle habit is "minimum or more" is 3 days.

Further, the control unit 43 determines the degree of achievement in the latest week (hereinafter, referred to as "one-week lifestyle habit achievement degree"). Specifically, the control unit 43 reads the degree of achievement of the lifestyle habit for the latest week from the usage log DB 423. Subsequently, the control unit 43 specifies the number of days in which the degree of achievement of the treatment menu is "achievement" in the latest week. The control unit 43 determines that the one-week lifestyle habit achievement degree is "not achieved" if the number of days in which the degree of achievement of the lifestyle habit is "achievement" is 0 days. Further, the control unit 43 determines that the one-week lifestyle habit achievement degree is "insufficient" if the number of days in which the degree of achievement of the lifestyle habit is "achievement" is 1 day to 5 days. Furthermore, the control unit 43 determines that the one-week lifestyle habit achievement degree is "minimum" if the number of days in which the degree of achievement of the lifestyle habit is "achievement" is 6 days to 7 days.

Then, the control unit 43 determines whether or not the disturbance of the lifestyle habit is recognized from the three-days lifestyle habit achievement degree and the one-week lifestyle habit achievement degree. Specifically, in a case where the three-days lifestyle habit achievement degree is "not achieved" or "insufficient", the control unit 43 assumes that a bad state continues if the one-week lifestyle habit achievement degree is also "not achieved" or "insufficient". Further, in the case, the control unit 43 assumes that the state is deteriorated even if the one-week lifestyle habit achievement degree is "minimum". As a result, the control unit 43 determines that the disturbance of the lifestyle habit is recognized. As described above, when the control unit 43 determines that the disengagement sign is not recognized in the patient, but the disturbance of the lifestyle habit is recognized therein, the control unit 43 determines that the balance of the treatment activity is deteriorated.

On the other hand, in a case where the three-days lifestyle habit achievement degree is "minimum", the control unit 43 assumes that the good state is continued if the one-week lifestyle habit achievement degree is "minimum". Further, in the case, the control unit 43 assumes that the state is improved even if the one-week lifestyle habit achievement degree is "insufficient". As a result, the control unit 43 determines that the disturbance of the lifestyle habit is not recognized. As described above, when the control unit 43 determines that neither the disengagement sign nor the disturbance of the lifestyle habit is recognized in the patient, the control unit 43 determines that the balance of the treatment activity is not deteriorated, and ends the balance determination process.

The control unit 43 reads the patient information and the supporter information corresponding to the patient identifier included in the usage log from the patient DB 421 when the control unit 43 determines that the disengagement sign is not recognized in the patient, but the disturbance of the lifestyle habit is recognized therein and thus the balance of the treatment activity is deteriorated. The control unit 43 detects, from the message DB 422, the plurality of types of messages corresponding to the characteristics of the patient included in the patient information read from the patient DB 421. Subsequently, the control unit 43 generates, from the plurality of types of messages detected from the message DB 422, the display data of the fourth message selection screen for selecting the message for supporting or encouraging the patient who is recognized as having the deterioration in the balance of the treatment activity. Then, the control unit 43 transmits the display data of the fourth message selection screen from the communication unit 41 via the network N to the supporter terminal 3-n possessed by the supporter identified from the supporter identifier included in the supporter information.

In response to receiving the fourth message selection command transmitted from the supporter terminal 3-n via the network N with the communication unit 41, the control unit 43 transmits the fourth message display command including the display name of the supporter identified from the supporter information corresponding to the supporter identifier included in the fourth message selection command and the message included in the fourth message selection command from the communication unit 41 to the patient terminal 2 via the network N.

### [Treatment activity determination process]

In the treatment activity determination process, first, the control unit 43 reads the degree of achievement of the treatment menu and the degree of achievement of the lifestyle habit corresponding to the latest date from the usage log DB 423. In addition, the control unit 43 reads the patient information and the supporter information corresponding to the patient identifier included in the usage log from the patient DB 421. The control unit 43 detects the plurality of types of messages corresponding to the characteristics of the patient included in the patient information read from the patient DB 421 from the message DB 422.

When the degree of achievement of either the treatment menu or the lifestyle habit on the day is "minimum" or "moderate", the control unit 43 generates, from the plurality of types of messages detected from the message DB 422, the display data of the fifth message selection screen for selecting the message for supporting the patient who is trying to do his/her best in the treatment activity. Then, the control unit 43 transmits the display data of the fifth message selection screen from the communication unit 41 via the network N to the supporter terminal 3-n possessed by the supporter identified from the supporter identifier included in the supporter information.

When the degrees of achievement of both of the treatment menu and the lifestyle habit on the day are "achievement", the control unit 43 generates, from the plurality of types of messages detected from the message DB 422, the display data of the sixth message selection screen for selecting the message for praising the patient who is sufficiently performing the treatment activity. Then, the control unit 43 transmits the display data of the sixth message selection screen from the communication unit 41 via the network N to the supporter terminal 3-n possessed by the supporter identified from the supporter identifier included in the supporter information.

In response to receiving the fifth or sixth message selection command transmitted from the supporter terminal 3-n via the network N with the communication unit 41, the control unit 43 transmits the fifth or sixth message display command including the display name of the supporter identified from the supporter information corresponding to the supporter identifier included in the fifth or sixth message selection command and the message included in the fifth or sixth message selection command from the communication unit 41 to the patient terminal 2 via the network N.

### [Second disengagement sign determination process]

Further, in response to receiving the answer content notification transmitted from the patient terminal 2 via the network N with the communication unit 41, the control unit 43 determines whether or not the disengagement sign is recognized in the patient by checking whether or not there is a negative answer such as "dissatisfied", "bored", and "reduced motivation" in the answer content to the questionnaire about the treatment activity included in the answer content notification. If the control unit 43 determines that there is no negative answer and thus no negative answer is recognized, the control unit 43 ends the second disengagement sign determination process as it is.

When the control unit 43 determines that there is at least one negative answer and thus the disengagement sign is recognized in the patient, the control unit 43 reads the patient information and the supporter information corresponding to the patient identifier included in the answer content notification from the patient DB 421. The control unit 43 detects, from the message DB 422, the plurality of types of messages corresponding to the characteristics of the patient included in the patient information read from the patient DB 421. Subsequently, the control unit 43 generates, from the plurality of types of messages detected from the message DB 422, the display data of the second message selection screen for selecting the message for encouraging or comforting the patient who is recognized as having the disengagement sign. Then, the control unit 43 transmits the display data of the second message selection screen from the communication unit 41 via the network N to the supporter terminal 3-n possessed by the supporter identified from the supporter identifier included in the supporter information.

In response to receiving the second message selection command transmitted from the supporter terminal 3-n via the network N with the communication unit 41, the control unit 43 transmits the second message display command including the display name of the supporter identified from the supporter information corresponding to the supporter identifier included in the second message selection command and the message included in the second message selection command from the communication unit 41 to the patient terminal 2 via the network N.

Next, the various processes executed by the treatment support system 1 having the above-described configuration will be described with reference to the drawings.

In response to the patient selecting a game to be played in the top screen of the treatment program displayed on the touch panel 22 of the patient terminal 2, the treatment support system 1 starts a play start process.

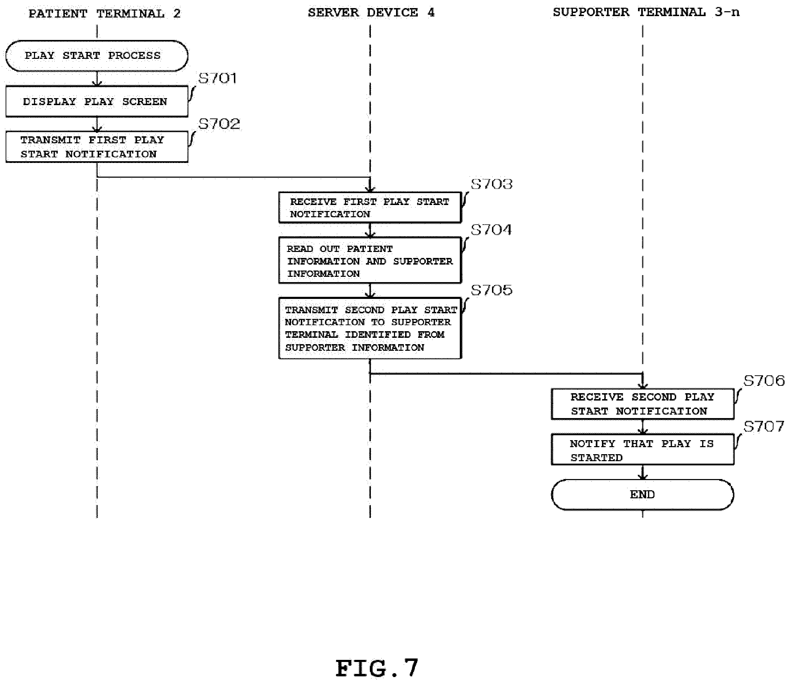

FIG. 7 is a flowchart showing details of a play start process.

In the play start process shown in FIG. 7, first, the control unit 24 of the patient terminal 2 causes the play screen 300 for playing the game to be displayed on the touch panel 22 (step S701).

In addition, the control unit 24 transmits, from the communication unit 23 to the server device 4 via the network N, the first play start notification including the patient identifier capable of identifying the patient who has started the game (step S702).

In response to receiving the first play start notification transmitted from the patient terminal 2 via the network N with the communication unit 41 (step S703), the control unit 43 of the server device 4 reads the patient information and the supporter information corresponding to the patient identifier included in the first play start notification from the patient DB 421 (step S704).

Next, the control unit 43 transmits the second play start notification from the communication unit 41 to the supporter terminal 3-n possessed by the supporter identified from the supporter information via the network N (step S705).

In response to receiving the second play start notification transmitted from the server device 4 via the network N with the communication unit 33 (step S706), the control unit 34 of the supporter terminal 3-n notifies the supporter that the patient has started playing the game (step S707), and then ends the play start process.

Thereafter, in response to the supporter tapping the icon indicating the support program displayed on the touch panel 32 and indicating to participate in the support for the patient playing the game, the control unit 34 of the supporter terminal 3-n activates the support program stored in the storage unit 31, and starts the first treatment support process in the treatment support system 1.

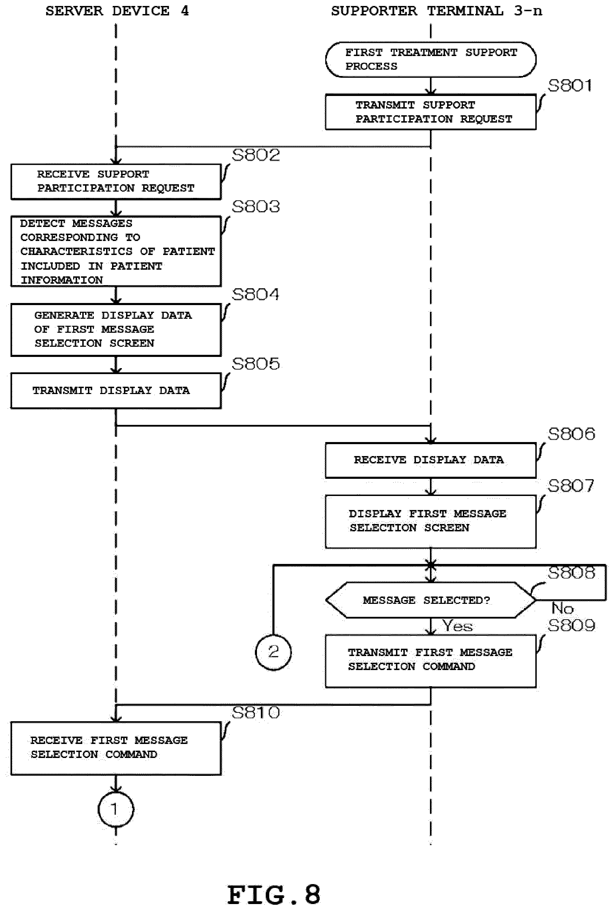

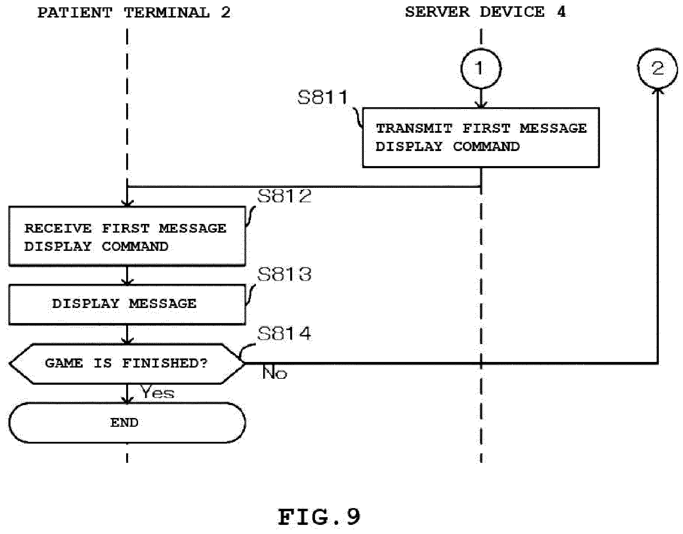

Each of FIG. 8 and FIG. 9 is a flowchart showing details of a first treatment support process.

In the first treatment support process shown in FIGs. 8 and 9, first, the control unit 34 transmits the support participation request including the supporter identifier capable of identifying the supporter from the communication unit 33 to the server device 4 via the network N (step S801 shown in FIG. 8).

In response to receiving the support participation request transmitted from the supporter terminal 3-n via the network N with the communication unit 41 (step S802), the control unit 43 of the server device 4 detects, from the message DB 422, the plurality of types of messages corresponding to the characteristics of the patient included in the patient information read from the patient DB 421 (step S803).

Subsequently, the control unit 43 generates the display data of the first message selection screen 500 for selecting a message for supporting the patient playing the game from among the plurality of types of messages detected from the message DB 422 (step S804).

Then, the control unit 43 transmits the display data of the first message selection screen 500 from the communication unit 41 via the network N to the supporter terminal 3-n possessed by the supporter identified from the supporter identifier included in the support participation request (step S805).

In response to receiving the display data of the first message selection screen 500 transmitted from the server device 4 via the network N with the communication unit 32 (step S806), the control unit 34 of the supporter terminal 3-n causes the first message selection screen 500 to be displayed on the touch panel 32 based on the display data (step S807).

Then, in response to the supporter tapping and selecting the message on the first message selection screen 500 (step S808; Yes), the control unit 34 transmits the first message selection command including the message selected by the supporter and the supporter identifier capable of identifying the supporter from the communication unit 32 via the network N (step S809).

In response to receiving the first message selection command transmitted from the supporter terminal 3-n via the network N with the communication unit 41 (step S810), the control unit 43 of the server device 4 transmits the first message display command including the display name of the supporter identified from the supporter information corresponding to the supporter identifier included in the first message selection command and the message included in the first message selection command from the communication unit 41 to the patient terminal 2 via the network N (step S811 shown in FIG. 9).

In response to receiving the first message display command transmitted from the server device 4 via the network N with the communication unit 23 (step S812), the control unit 24 of the patient terminal 2 causes the display name of the supporter identified from the first message display command and the message selected by the supporter to be displayed on the play screen 300 below the game being played by the patient (step S813).

Until the patient finishes playing the game (step S814; No), the control unit 24 returns to the step S808 and waits for the supporter to select a new message on the first message selection screen 500. Then, when the patient finishes playing the game (step S814; No), the control unit 24 ends the first treatment support process.

In response to the predetermined transmission timing (e.g., 12 PM, etc.) of the day, the treatment support system 1 starts the second treatment support process.

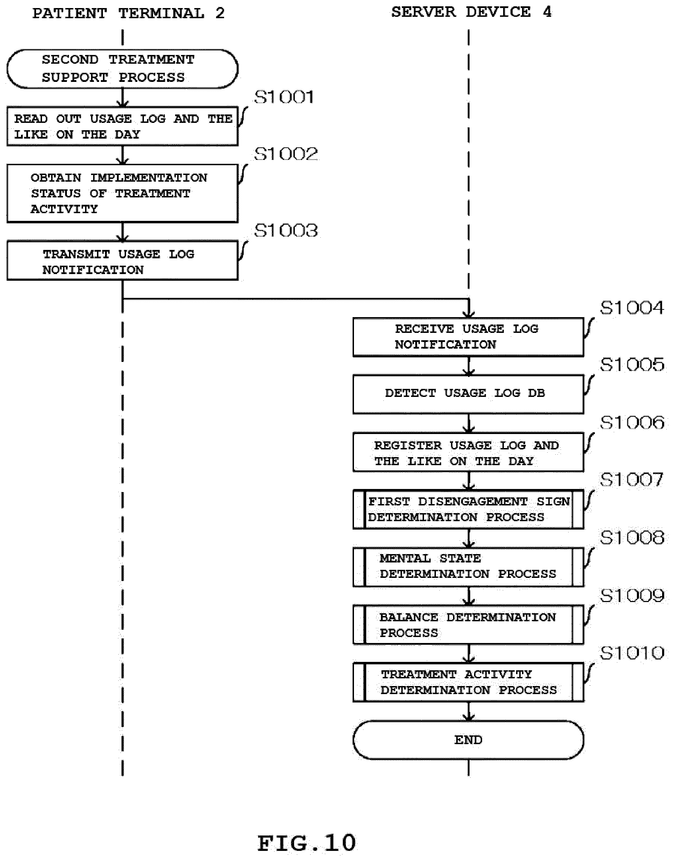

FIG. 10 is a flowchart showing details of a second treatment support process.

In the second treatment support process shown in FIG. 10, first, the control unit 24 of the patient terminal 2 reads, from the storage unit 21, the usage log such as the usage time and the number of times of activation of the treatment program on the day, the number of times of answer to the questionnaire about the lifestyle habit, the content of answer, the patient viewpoint mental log, and the like (step S1001).

Next, the control unit 24 obtains the implementation status of the treatment activity from the number of times of activation of the treatment program, the number of times of answer to the questionnaire about the lifestyle habit, and the content of the answer (step S1002).

Then, the control unit 24 transmits, from the communication unit 23 to the server device 4 via the network N, the usage log notification including the usage log such as the patient identifier, the date, the usage time and the number of times of activation of the treatment program, and the implementation status of the treatment activity (step S1003).

In response to receiving the usage log notification transmitted from the patient terminal 2 via the network N with the communication unit 41 (step S1004), the control unit 43 of the server device 4 detects the usage log DB 423 of the patient identified from the patient identifier included in the usage log notification from the storage unit 42 (step S1005).

Subsequently, the control unit 43 correspondingly registers the date, the usage log such as the usage time and the number of times of activation of the treatment program, the implementation status of the treatment activity, the patient viewpoint mental log, and the like included in the usage log notification in the usage log DB 423 detected from the storage unit 42 (step S1006).

Then, the control unit 43 executes the first disengagement sign determination process (step S1007), the mental state determination process (step S1008), the balance determination process (step S1009), and the treatment activity determination process (step S1010) on the basis of the usage log such as the usage time and the number of times of activation of the treatment program, the implementation status of the treatment activity, the patient viewpoint mental log registered in the usage log DB 423, the supporter viewpoint mental log registered in the mental log DB 424, and the like.

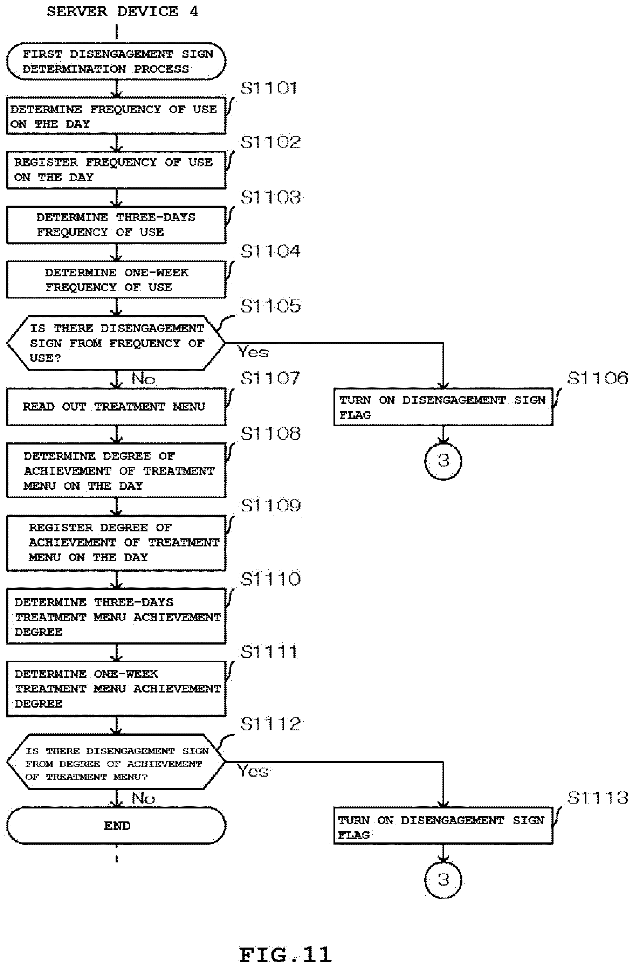

Each of FIG. 11 to FIG. 13 is a flowchart showing details of a first disengagement sign determination process.

In the first disengagement sign determination process shown in FIGs. 11 to 13, first, the control unit 43 determines the frequency of use on the day from the usage log such as the usage time and the number of times of activation of the treatment program corresponding to the latest date (step S1101 shown in FIG. 11).

Then, the control unit 43 registers the frequency of use of the treatment program in the usage log DB 423 in association with the latest date (step S1102).

Next, the control unit 43 determines three-days frequency of use (step S1103).

Further, the control unit 43 determines the one-week frequency of use (step S1104).

Then, the control unit 43 determines whether or not there is the disengagement sign in the patient from the three-days frequency of use and the one-week frequency of use (step S1105).

When the control unit 43 determines that the disengagement sign is recognized in the patient from the frequency of use of the treatment program (step S1105; Yes), the control unit 43 turns on the disengagement sign flag (step S1106).

On the other hand, if the control unit 43 determines, from the frequency of use of the treatment program, that the disengagement sign is not recognized in the patient (step S1105; No), the control unit 43 reads the treatment menu corresponding to the patient identifier included in the usage log from the patient DB 421 (step S1107).

The control unit 43 compares the implementation status of the treatment activity corresponding to the latest date with the treatment menu read from the patient DB 421 to determine the degree of achievement by obtaining the achievement rate of the treatment menu (step S1108).

Then, the control unit 43 registers the degree of achievement of the treatment menu in the usage log DB 423 in association with the latest date (step S1109).

Next, the control unit 43 determines the three-days treatment menu achievement degree (step S1110).

Further, the control unit 43 determines the one-week treatment menu achievement degree (step S1111).

Then, the control unit 43 determines whether or not the disengagement sign is recognized in the patient from the three-days treatment menu achievement degree and the one-week treatment menu achievement degree (step S1112).

If the control unit 43 determines that the disengagement sign is recognized in the patient from the degree of achievement of the treatment menu (step S1112; Yes), the control unit 43 turns on the disengagement sign flag (step S1113).

On the other hand, if the control unit 43 determines that the disengagement sign is not recognized in the patient (step S1112; No) based on not only the frequency of use of the treatment program but also the degree of achievement of the treatment menu, the control unit 43 ends the first disengagement sign determination process as it is.

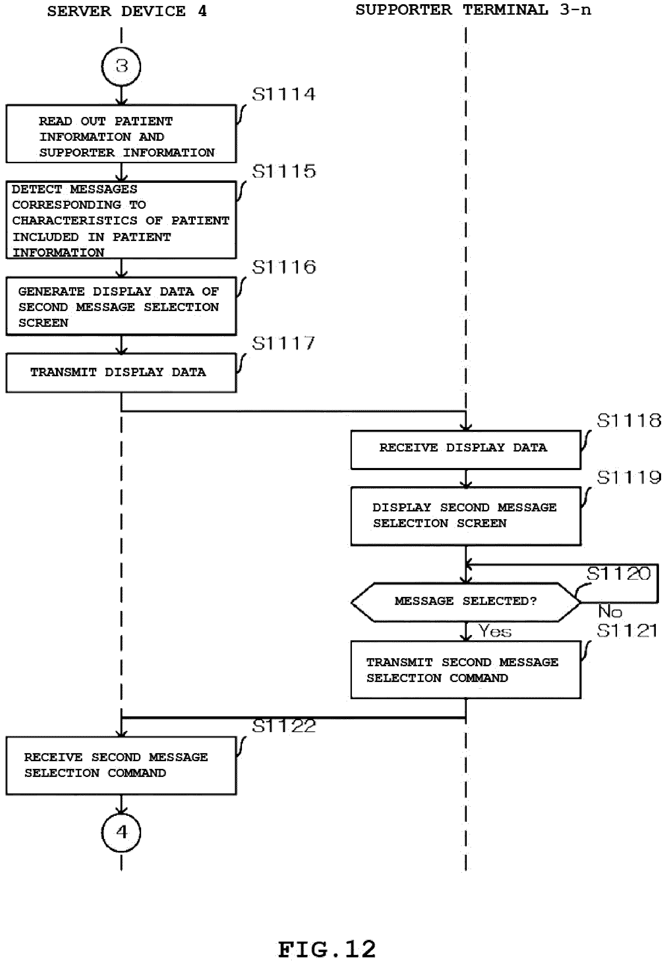

When the control unit 43 determines that the disengagement sign is recognized in the patient (step S1105; Yes, step S1112; Yes), the control unit 43 reads the patient information and the supporter information corresponding to the patient identifier included in the usage log from the patient DB 421 (step S1114 shown in FIG. 12).

The control unit 43 detects, from the message DB 422, the plurality of types of messages corresponding to the characteristics of the patient included in the patient information read from the patient DB 421 (step S1115).

Subsequently, the control unit 43 generates, from the plurality of types of messages detected from the message DB 422, the display data of the second message selection screen for selecting the message for encouraging or comforting the patient who is recognized as having the disengagement sign (step S1116).

Then, the control unit 43 transmits the display data of the second message selection screen from the communication unit 41 via the network N to the supporter terminal 3-n possessed by the supporter identified from the supporter identifier included in the supporter information (step S1117).

In response to receiving the display data of the second message selection screen transmitted from the server device 4 via the network N with the communication unit 32 (step S1118), the control unit 34 of the supporter terminal 3-n causes the second message selection screen to be displayed on the touch panel 32 based on the display data (step S1119).

Then, in response to the supporter tapping and selecting the message on the second message selection screen (step S1120; Yes), the control unit 34 transmits the second message selection command including the message selected by the supporter and the supporter identifier capable of identifying the supporter from the communication unit 32 via the network N (step S1121).

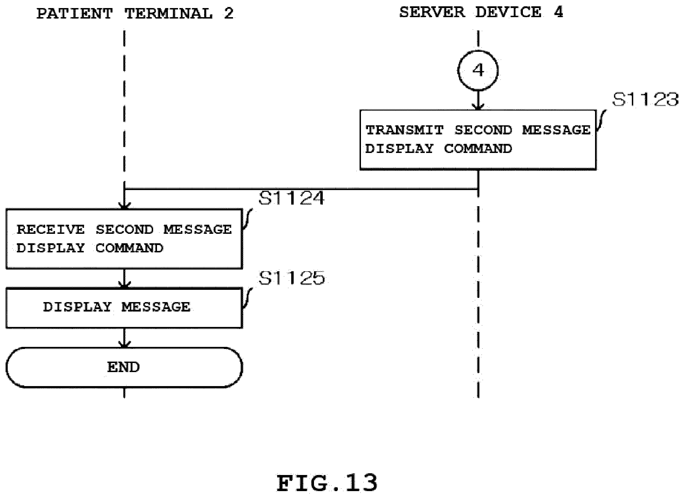

In response to receiving the second message selection command transmitted from the supporter terminal 3-n via the network N with the communication unit 41 (step S1122), the control unit 43 of the server device 4 transmits the second message display command including the display name of the supporter identified from the supporter information corresponding to the supporter identifier included in the second message selection command and the message included in the second message selection command from the communication unit 41 to the patient terminal 2 via the network N (step S1123 shown in FIG. 13).

In response to receiving the second message display command transmitted from the server device 4 via the network N with the communication unit 23 (step S1124), the control unit 24 of the patient terminal 2 causes the display name of the supporter identified from the second message display command and the message selected by the supporter to be displayed on the touch panel 22 (step S1125), and then ends the first disengagement sign determination process.

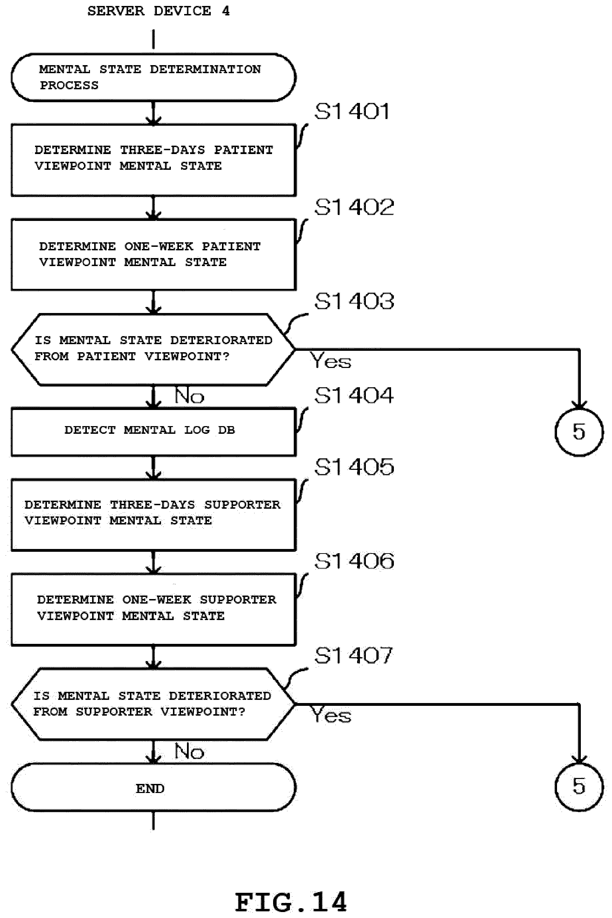

Each of FIG. 14 to FIG. 16 is a flowchart showing details of the mental state determination process.

In the mental state determination process shown in FIGs. 14 to 16, first, the control unit 43 of the server device 4 determines the three-days patient viewpoint mental state (step S1401 shown in FIG. 14).

Next, the control unit 43 determines the one-week patient viewpoint mental state (step S1402).

Then, the control unit 43 determines whether or not the deterioration of the mental state is recognized in the patient from the viewpoint of the patient based on the three-days patient viewpoint mental state and the one-week patient viewpoint mental state (step S1403).

If the control unit 43 determines, from the patient viewpoint, that the deterioration of the mental state is not recognized in the patient (step S1403; No), the control unit 43 detects, from the storage unit 42, the mental log DB 424 of the supporter identified from the supporter identifier included in the supporter viewpoint mental log (step S1404).

Then, the control unit 43 determines the three-days supporter viewpoint mental state (step S1405).

Next, the control unit 43 determines the one-week supporter viewpoint mental state (step S1406).

Then, the control unit 43 determines whether or not the deterioration of the mental state is recognized in the patient from the viewpoint of the supporter based on the three-days supporter viewpoint mental state and the one-week supporter viewpoint mental state (step S1407).

If the control unit 43 determines that the deterioration of the mental state is not recognized in the patient not only from the patient viewpoint but also from the supporter viewpoint (step S1407; No), the control unit 43 ends the mental state determination process as it is.

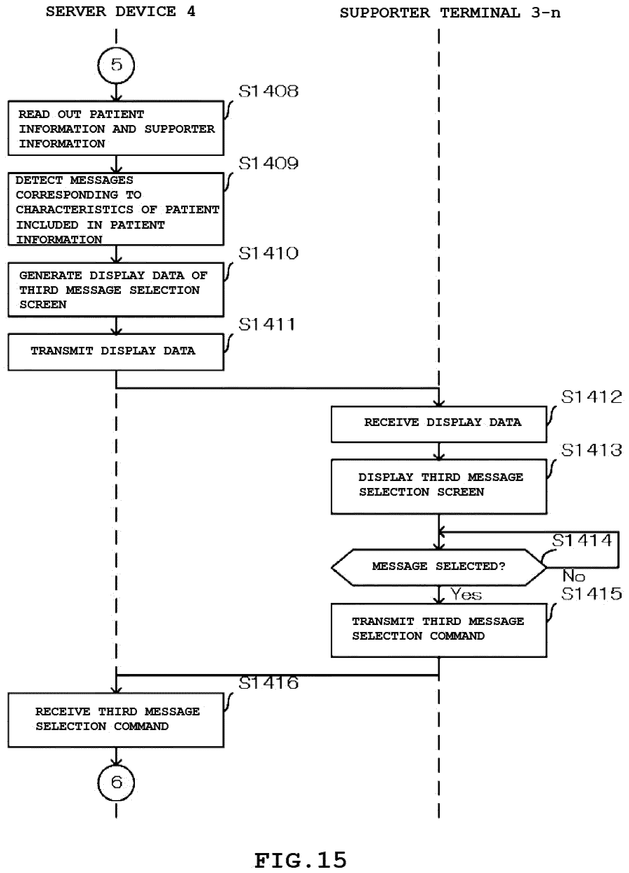

When it is determined that the mental state of the patient is deteriorated from either the patient viewpoint or the supporter viewpoint (step S1403; Yes, step S1407; Yes), the control unit 43 reads the patient information and the supporter information corresponding to the patient identifier included in the usage log from the patient DB 421 (step S1408 shown in FIG. 15).

The control unit 43 detects, from the message DB 422, the plurality of types of messages corresponding to the characteristics of the patient included in the patient information read from the patient DB 421 (step S1409).

Subsequently, the control unit 43 generates, from the plurality of types of messages detected from the message DB 422, the display data of the third message selection screen for selecting the message for encouraging or comforting the patient who is recognized to be deteriorated in the mental state (step S1410).

Then, the control unit 43 transmits the display data of the third message selection screen from the communication unit 41 via the network N to the supporter terminal 3-n possessed by the supporter identified from the supporter identifier included in the supporter information (step S141 1).

In response to receiving the display data of the third message selection screen transmitted from the server device 4 via the network N with the communication unit 32 (step S1412), the control unit 34 of the supporter terminal 3-n causes the third message selection screen to be displayed on the touch panel 32 based on the display data (step S1413).

Then, in response to the supporter tapping and selecting the message on the third message selection screen (step S1414; Yes), the control unit 34 transmits the third message selection command including the message selected by the supporter and the supporter identifier capable of identifying the supporter from the communication unit 32 via the network N (step S1415).

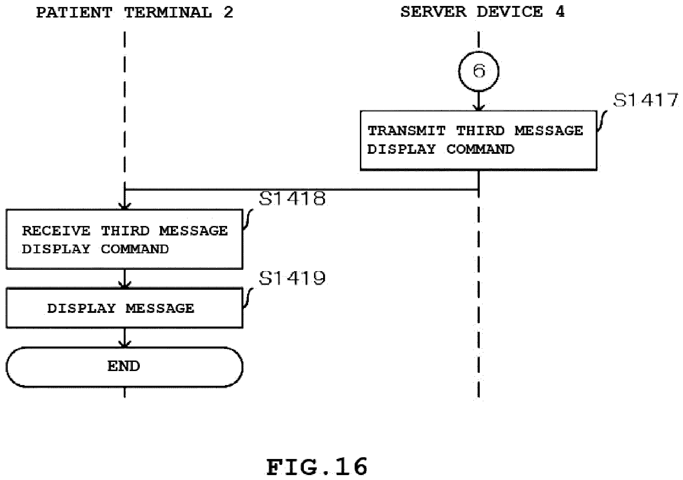

In response to receiving the third message selection command transmitted from the supporter terminal 3-n via the network N with the communication unit 41 (step S1416), the control unit 43 of the server device 4 transmits the third message display command including the display name of the supporter identified from the supporter information corresponding to the supporter identifier included in the third message selection command and the message included in the second message selection command from the communication unit 41 to the patient terminal 2 via the network N (step S1417 shown in FIG. 16).

In response to receiving the third message display command transmitted from the server device 4 via the network N with the communication unit 23 (step S1418), the control unit 24 of the patient terminal 2 causes the display name of the supporter identified from the third message display command and the message selected by the supporter to be displayed on the touch panel 22 (step S1419), and then ends the mental state determination process.

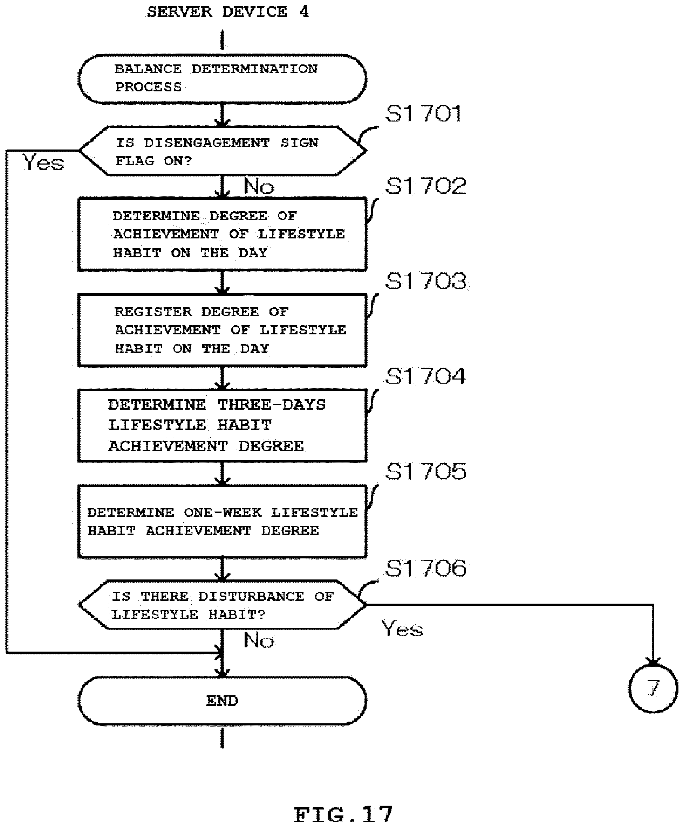

Each of FIG. 17 to FIG. 19 is a flowchart showing details of the balance determination process.

In the balance determination process shown in FIGs. 17 to 19, first, the control unit 43 of the server device 4 determines whether or not the disengagement sign flag is on (step S1701 shown in FIG. 17).

When the disengagement sign flag is on (step S1701; Yes), the control unit 43 determines that the disengagement sign is recognized in the patient, and ends the balance determination process as it is.

On the other hand, when the disengagement sign flag is off (step S1701; No), that is, when the disengagement sign is not recognized in the patient, the control unit 43 determines the degree of achievement of the lifestyle habit by obtaining the achievement rate of the lifestyle habit from the number of times of answer and the content of answer to the questionnaire about the lifestyle habit included in the implementation status of the treatment activity corresponding to the latest date (step S1702).

Then, the control unit 43 registers the degree of achievement of the lifestyle habit of the patient in the usage log DB 423 in association with the latest date (step S1703).

Next, the control unit 43 determines the three-days lifestyle habit achievement degree (step S1704).

Further, the control unit 43 determines the one-week lifestyle habit achievement degree (step S1705).

Then, the control unit 43 determines whether or not the disturbance of the lifestyle habit is recognized from the three-days lifestyle habit achievement degree and the one-week lifestyle habit achievement degree (step S1706).

If the control unit 43 determines that neither the disengagement sign nor the disturbance of the lifestyle habit is recognized in the patient (step S1706; No), the control unit 43 determines that the deterioration in the balance of the treatment activity is not recognized, and ends the balance determination process.

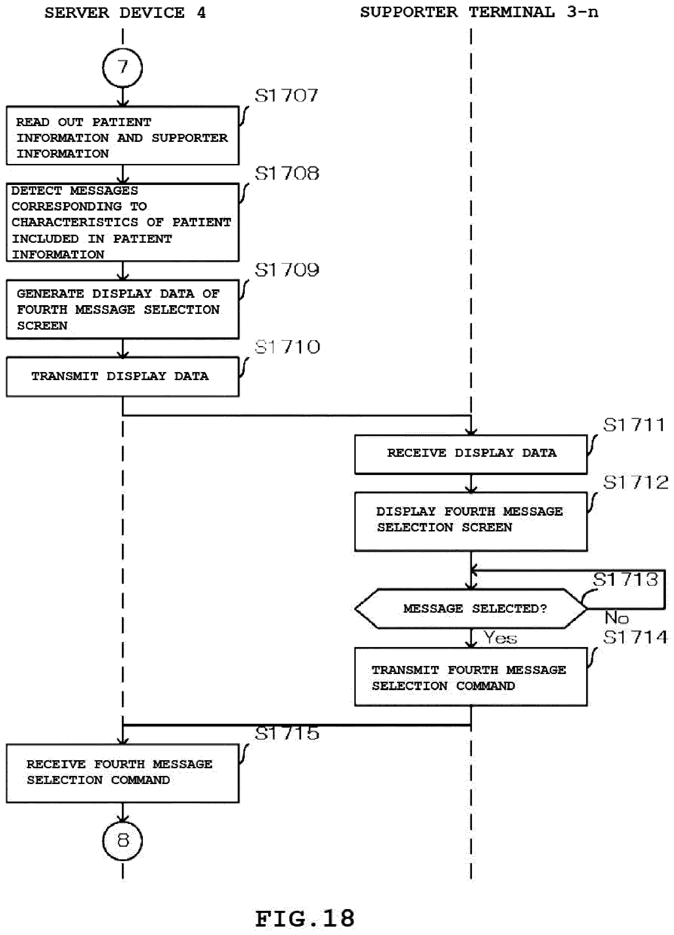

On the other hand, when the control unit 43 determines that the disengagement sign is not recognized in the patient, but the disturbance of the lifestyle habit is recognized in the patient, and thus the deterioration in the balance of the treatment activity is recognized (step S1706; Yes), the control unit 43 reads the patient information and the supporter information corresponding to the patient identifier included in the usage log from the patient DB 421 (step S1707 shown in FIG. 18).

The control unit 43 detects, from the message DB 422, the plurality of types of messages corresponding to the characteristics of the patient included in the patient information read from the patient DB 421 (step S1708).

Subsequently, the control unit 43 generates the display data of the fourth message selection screen for selecting the message for supporting or encouraging the patient whose treatment activity is recognized to be deteriorated from the plurality of types of messages detected from the message DB 422 (step S1709).

Then, the control unit 43 transmits the display data of the fourth message selection screen from the communication unit 41 via the network N to the supporter terminal 3-n possessed by the supporter identified from the supporter identifier included in the supporter information (step S1710).

In response to receiving the display data of the fourth message selection screen transmitted from the server device 4 via the network N with the communication unit 32 (step S1711), the control unit 34 of the supporter terminal 3-n causes the fourth message selection screen to be displayed on the touch panel 32 based on the display data (step S1712).

Then, in response to the supporter tapping and selecting the message on the fourth message selection screen (step S1713; Yes), the control unit 34 transmits the fourth message selection command including the message selected by the supporter and the supporter identifier capable of identifying the supporter from the communication unit 32 via the network N (step S1714).

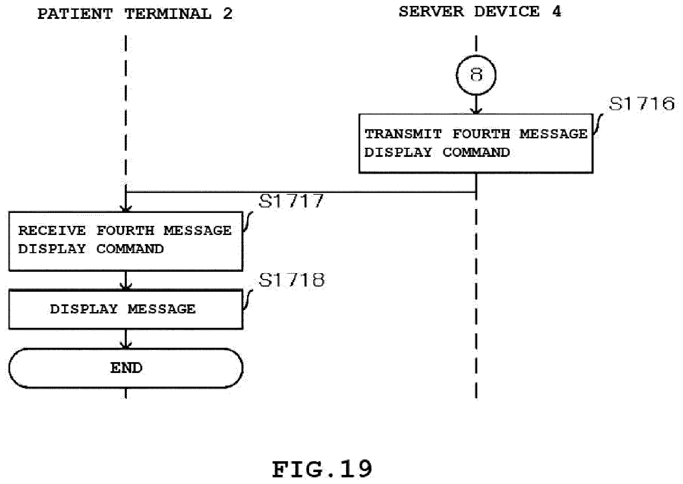

In response to receiving the fourth message selection command transmitted from the supporter terminal 3-n via the network N with the communication unit 41 (step S1715), the control unit 43 of the server device 4 transmits the fourth message display command including the display name of the supporter identified from the supporter information corresponding to the supporter identifier included in the fourth message selection command and the message included in the fourth message selection command from the communication unit 41 to the patient terminal 2 via the network N (step S1716 shown in FIG. 19).

In response to receiving the fourth message display command transmitted from the server device 4 via the network N with the communication unit 23 (step S1717), the control unit 24 of the patient terminal 2 causes the display name of the supporter identified from the fourth message display command and the message selected by the supporter to be displayed on the touch panel 22 (step S1718), and then ends the balance determination process.

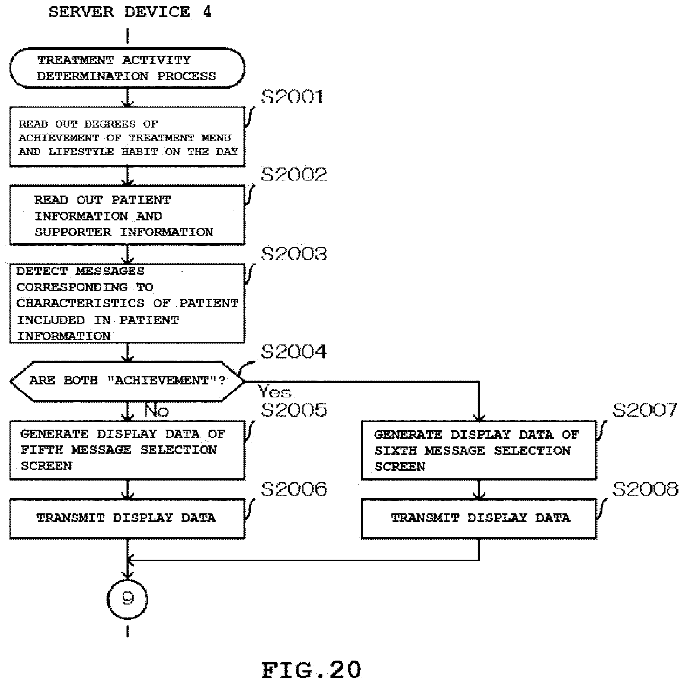

Each of FIG. 20 to FIG. 22 is a flowchart showing details of the treatment activity determination process.

In the treatment activity determination process shown in FIGs. 20 to 22, first, the control unit 43 reads the degrees of achievement of the treatment menu and the lifestyle habit corresponding to the latest date from the usage log DB 423 (step S2001 shown in FIG. 20).

Further, the control unit 43 reads the patient information and the supporter information corresponding to the patient identifier included in the usage log from the patient DB 421 (step S2002).

The control unit 43 detects, from the message DB 422, the plurality of types of messages corresponding to the characteristics of the patient included in the patient information read from the patient DB 421 (step S2003).

When the degree of achievement of either the treatment menu or the lifestyle habit on the day is "minimum" or "moderate" (step S2004; No), the control unit 43 generates the display data of the fifth message selection screen for selecting the message for supporting the patient who is trying to do his/her best in the treatment activity from the plurality of types of messages detected from the message DB 422 (step S2005).

Then, the control unit 43 transmits the display data of the fifth message selection screen from the communication unit 41 via the network N to the supporter terminal 3-n possessed by the supporter identified from the supporter identifier included in the supporter information (step S2006).

When the degrees of achievement of both of the treatment menu and the lifestyle habit on the day are "achievement" (step S2004; Yes), the control unit 43 generates the display data of the sixth message selection screen for selecting the message for praising the patient who is sufficiently performing the treatment activity from the plurality of types of messages detected from the message DB 422 (step S2007).

Then, the control unit 43 transmits the display data of the sixth message selection screen from the communication unit 41 via the network N to the supporter terminal 3-n possessed by the supporter identified from the supporter identifier included in the supporter information (step S2008).

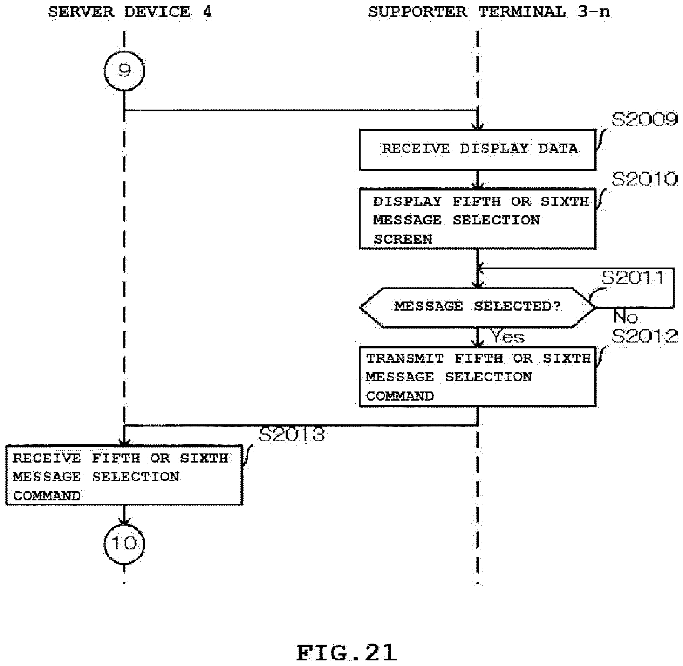

In response to receiving the display data of the fifth or sixth message selection screen transmitted from the server device 4 via the network N with the communication unit 32 (step S2009 shown in FIG. 21), the control unit 34 of the supporter terminal 3-n causes the fifth or sixth message selection screen to be displayed on the touch panel 32 based on the display data (step S2010).

Then, in response to the supporter tapping and selecting the message on the fifth or sixth message selection screen (step S2011; Yes), the control unit 34 transmits the fifth or sixth message selection command including the message selected by the supporter and the supporter identifier capable of identifying the supporter from the communication unit 32 via the network N (step S2012).

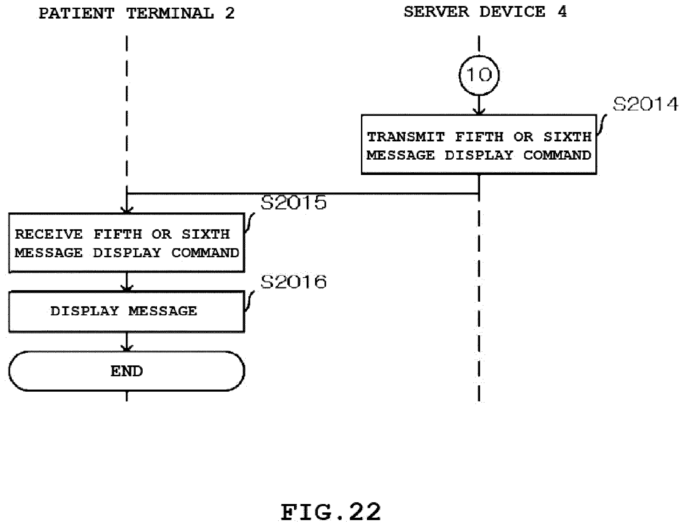

In response to receiving the fifth or sixth message selection command transmitted from the supporter terminal 3-n via the network N with the communication unit 41 (step S2013), the control unit 43 of the server device 4 transmits the fifth or sixth message display command including the display name of the supporter identified from the supporter information corresponding to the supporter identifier included in the fifth or sixth message selection command and the message included in the fifth or sixth message selection command from the communication unit 41 to the patient terminal 2 via the network N (step S2014 shown in FIG. 22).

In response to receiving the fifth or sixth message display command transmitted from the server device 4 via the network N with the communication unit 23 (step S2015), the control unit 24 of the patient terminal 2 causes the display name of the supporter identified from the fifth or sixth message display command and the message selected by the supporter to be displayed on the touch panel 22 (step S2016), and then ends the treatment activity determination process.

Further, in response to the patient inputting the answer content to the questionnaire about the treatment activity on the touch panel 22 of the patient terminal 2, the treatment support system 1 starts the second disengagement sign determination process.

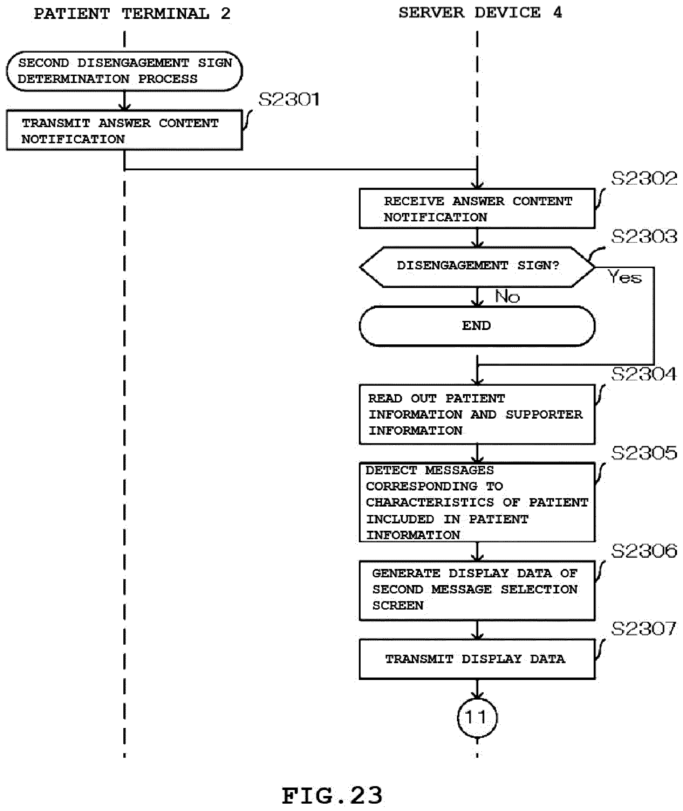

Each of FIG. 23 to FIG. 25 is a flowchart showing details of a second disengagement sign determination process.

In the second disengagement sign determination process shown in FIGs. 23 to 25, the control unit 24 of the patient terminal 2 transmits the answer content notification including the patient identifier, the answer content to the questionnaire about the treatment activity and the like from the communication unit 23 to the server device 4 via the network N (step S2301 shown in FIG. 23).

In response to receiving the answer content notification transmitted from the patient terminal 2 via the network N with the communication unit 41 (step S2302), the control unit 43 of the server device 4 checks whether or not there is the negative response such as "dissatisfied", "bored", and "reduced motivation" in the answer content to the questionnaire about the treatment activity included in the answer content notification, thereby determining whether or not the disengagement sign is recognized in the patient (step S2303).

When the control unit 43 determines that there is no negative answer and thus no disengagement sign is recognized in the patient (step S2303; No), the control unit 43 ends the second disengagement sign determination process as it is.

When the control unit 43 determines that there is at least one negative answer and thus the disengagement sign is recognized in the patient (step S2303; Yes), the control unit 43 reads the patient information and the supporter information corresponding to the patient identifier included in the answer content notification from the patient DB 421 (step S2304).

The control unit 43 detects, from the message DB 422, the plurality of types of messages corresponding to the characteristics of the patient included in the patient information read from the patient DB 421 (step S2305).

Subsequently, the control unit 43 generates, from the plurality of types of messages detected from the message DB 422, the display data of the second message selection screen for selecting the message for encouraging or comforting the patient who is recognized as having the disengagement sign (step S2306).

Then, the control unit 43 transmits the display data of the second message selection screen from the communication unit 41 via the network N to the supporter terminal 3-n possessed by the supporter identified from the supporter identifier included in the supporter information (step S2307).

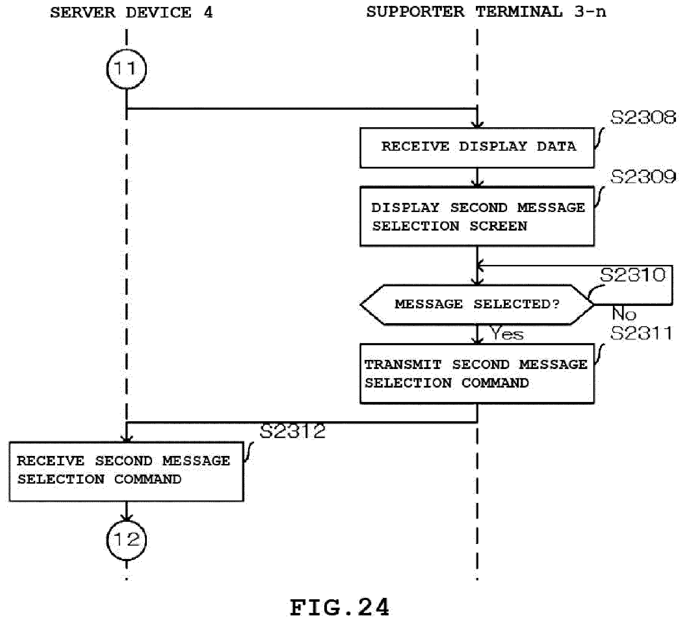

In response to receiving the display data of the second message selection screen transmitted from the server device 4 via the network N with the communication unit 32 (step S2308 shown in FIG. 24), the control unit 34 of the supporter terminal 3-n causes the second message selection screen to be displayed on the touch panel 32 based on the display data (step S2309).

Then, in response to the supporter tapping and selecting the message on the second message selection screen (step S2310; Yes), the control unit 34 transmits the second message selection command including the message selected by the supporter and the supporter identifier capable of identifying the supporter from the communication unit 32 via the network N (step S2311).

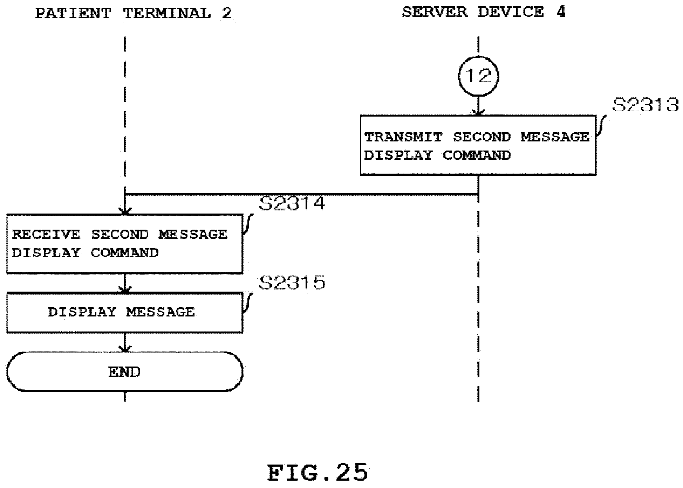

In response to receiving the second message selection command transmitted from the supporter terminal 3-n via the network N with the communication unit 41 (step S2312), the control unit 43 of the server device 4 transmits the second message display command including the display name of the supporter identified from the supporter information corresponding to the supporter identifier included in the second message selection command and the message included in the second message selection command from the communication unit 41 to the patient terminal 2 via the network N (step S2313 shown in FIG. 25).

In response to receiving the second message display command transmitted from the server device 4 via the network N with the communication unit 23 (step S2314), the control unit 24 of the patient terminal 2 causes the display name of the supporter identified from the second message display command and the message selected by the supporter to be displayed on the touch panel 22 (step S2315), and then ends the second disengagement sign determination process.

As described above, the treatment support system 1 according to the present embodiment includes the patient terminal 2 used by the patient for the training for the treatment, the supporter term inal 3-n of the supporter of the patient, and the server device 4 connected to these terminals via the network N and including the patient DB 421 and the message DB 422. The patient DB 421 registers, for each patient, the characteristics of the patient and the supporter information capable of identifying the supporter terminal 3-n of the supporter of the patient in association with each other. The message DB 422 registers the messages effective for the treatment of the patient in association with the characteristics of the patient.

When the predetermined condition is satisfied, the control unit (server control unit) 43 of the server device 4 transmits the display data for displaying, in a selectable manner, the messages registered in the message DB 422 to the supporter terminal 3-n via the network N.

The supporter terminal 3-n includes the display unit 32 for displaying the messages effective for the treatment of the patient in a selectable manner based on the display data in response to receiving the display data transmitted from the server device 4 via the network N. The control unit (terminal control unit) 34 of the supporter terminal 3-n transmits the message selection command indicating the message selected by the supporter from among the messages displayed on the display unit 32 to the server device 4 via the network N.

In response to receiving the message selection command transmitted from the supporter terminal 3-n via the network N, the control unit 43 of the server device 4 causes the message specified from the message selection command to be displayed on the patient terminal 2 via the network N as the message from the supporter.

For example, in response to receiving the usage history (usage log notification) of the patient terminal 2 via the network N, the control unit 43 determines whether or not the disengagement sign from the training is recognized in the patient based on the usage history of the patient terminal 2, more specifically, based on the usage log such as the usage time and the number of times of activation of the treatment program. If the control unit 43 determines that the disengagement sign is recognized in the patient, assuming that the predetermined condition is satisfied, the control unit 43 causes the messages registered in the message DB 422 to be displayed on the supporter terminal 3-n via the network N in a selectable manner.

More specifically, the control unit 43 specifies the frequency of use of the treatment program for treating the patient installed in the patient terminal 2 from the usage history of the patient terminal 2. Then, when the frequency of use of the treatment program does not satisfy the predetermined standard, the control unit 43 determines that the disengagement sign from the training is recognized in the patient.

On the other hand, when the frequency of use of the treatment program satisfies the predetermined standard, the control unit 43 specifies the degree of achievement of the treatment menu of the patient from the usage history of the patient terminal 2. Then, when the frequency of use of the treatment program satisfies the predetermined standard, but the degree of achievement of the treatment menu does not satisfy the predetermined standard, the control unit 43 determines that the disengagement sign from the training is recognized in the patient.

Further, in response to receiving the answer to the questionnaire about the lifestyle habit via the network N, the answer input by the patient into the patient terminal 2, if the control unit 43 determines that the disengagement sign is not recognized in the patient, the control unit 43 determines whether or not the disturbance of the lifestyle habit is recognized in the patient from the answer to the questionnaire about the lifestyle habit. If the control unit 43 determines that the disturbance of the lifestyle habit is recognized in the patient, assuming that the predetermined condition is satisfied, the control unit 43 causes the messages registered in the message DB 422 to be displayed on the supporter terminal 3-n via the network N in a selectable manner.

Further, in response to receiving the answer to the questionnaire about the patient's mood via the network N, the answer input by the patient into the patient terminal 2, the control unit 43 determines whether or not the deterioration of the mental state is recognized in the patient from the viewpoint of the patient based on the answer to the questionnaire about the patient's mood. Then, if the control unit 43 determines that the deterioration of the mental state is recognized in the patient from the viewpoint of the patient, assuming that the predetermined condition is satisfied, the control unit 43 causes the messages registered in the message DB 422 to be displayed on the supporter terminal 3-n via the network N in a selectable manner.

Further, in response to receiving the answer to the questionnaire about the patient's mood via the network N, the answer input by the supporter into the supporter terminal 3-n, the control unit 43 determines whether or not the deterioration of the mental state is recognized in the patient from the viewpoint of the supporter based on the answer to the questionnaire about the patient's mood. If the control unit 43 determines that the deterioration of the mental state is recognized in the patient from either of the viewpoint of the patient or the viewpoint of the supporter, assuming that the predetermined condition is satisfied, the control unit 43 causes the messages registered in the message DB 422 to be displayed on the supporter terminal 3-n via the network N in a selectable manner.

Further, in response to receiving the usage history of the patient terminal 2 and the answer to the questionnaire about the lifestyle via the network N, the answer input by the patient into the patient terminal 2, the control unit 43 specifies the degree of achievement of the treatment menu of the patient from the usage history of the patient terminal 2, and specifies the degree of achievement of the lifestyle habit of the patient from the answer to the questionnaire about the lifestyle habit. Then, when both of the treatment menu and the lifestyle habit satisfy the predetermined standard, the control unit 43 causes the messages for praising the patient among the messages registered in the message DB 422 to be displayed on the supporter terminal 3-n via the network N in a selectable manner. Further, when the treatment menu or the lifestyle habit does not satisfy the predetermined standard, the control unit causes the messages for supporting the patient among the messages registered in the message DB 422 to be displayed on the supporter terminal 3-n via the network N in a selectable manner.

Further, in response to receiving the answer to the questionnaire about the treatment activity via the network N, the answer input by the patient into the patient terminal 2, the control unit 43 determines whether or not the disengagement sign from the training is recognized in the patient from the answer to the questionnaire about the treatment activity. If the control unit 43 determines that the disengagement sign is recognized in the patient, assuming that the predetermined condition is satisfied, the control unit 43 causes the messages registered in the message DB 422 to be displayed on the supporter terminal 3-n via the network N in a selectable manner.

As described above, in the present embodiment, only the messages effective for the treatment of the patient with MCI are prepared in advance in the message DB 422 under the supervision of the experts who have relevant knowledge of the treatment of the patient with MCI. Thus, the supporter can autonomously and safely interact with the patient to effectively support the patient by selecting, from the message DB 422, the message for supporting, encouraging and comforting the patient who is recognized as having the disengagement sign, the deterioration of the mental state and the disturbance of the lifestyle habit, and the patient who is trying to do his/her best in the treatment activity, or the message for praising the patient who is sufficiently performing the treatment activity. This can prevent the deterioration of the symptoms of the patient with MCI. This also allows early detection of symptoms in the patient with MCI to handle them. Furthermore, the mental and physical health of the supporter is improved by providing "psychological support" such as reducing the stress of the supporter (family, etc.) other than the patient. As a result, it is also possible to contribute to the improvement of the therapeutic effect of the patient himself/herself.

According to the treatment support system 1 of the present embodiment, while a therapist and the patient are in a normal treatment relationship, the patient and a third party such as the family or the supporter are connected with an Information and Communication Technology (ICT). Thereby, effects such as improvement of deterioration of the symptoms, enhancement of the therapeutic effect, improvement of adherence, improvement of treatment continuity, improvement of Quality of Life (QOL) of the patient and the third party such as the family or the supporter, reduction of anxiety, learning effect by education on diseases, and the like can be expected for all diseases, not limited to MCI.

Further, the control unit (server control unit) 43 of the server device 4 reads the characteristics of the patient and the supporter information from the patient DB 421 in response to receiving the notification of the start of the training (play of the game) of the patient by the first play start notification transmitted from the patient terminal 2 via the network N. Then, the control unit 43 transmits the second play start notification via the network N to the supporter terminal 3-n identified from the supporter information read from the patient DB 421, thereby notifying the start of the training of the patient to the supporter terminal 3-n of the supporter of the patient. After the start of the training of the patient is notified, the supporter terminal 3-n transmits the support participation request to the server device 4 via the network N in response to the supporter indicating to participate in the support of the training of the patient (support of the patient playing the game).

Thereafter, the control unit 43 detects, from the message DB 422, the messages corresponding to the characteristics of the patient read from the patient DB 421 in response to the indication to participate in the support of the training of the patient by the support participation request transmitted from the supporter terminal 3-n via the network N. Then, the control unit 43 transmits the display data for displaying the messages detected from the message DB 422 in a selectable manner to the supporter terminal 3-n identified from the supporter information read from the patient DB 421, thereby causing the messages effective for the treatment of the patient to be displayed in a selectable manner on the supporter terminal 3-n indicating to participate in the support of the training of the patient.

In response to receiving the display data transmitted from the server device 4 via the network N, the supporter terminal 3-n displays the messages corresponding to the characteristics of the patient and effective for the treatment of the patient on the display unit 32 in a selectable manner based on the display data. In addition, the display unit 32 displays the state of the training of the patient. Then, the control unit (terminal control unit) 34 of the supporter terminal 3-n transmits the message selection command indicating the message selected by the supporter from the messages displayed on the display unit 32 to the server device 4 via the network N.

In response to receiving the message selection command transmitted from the supporter terminal 3-n via the network N, the control unit 43 of the server device 4 causes the message specified from the message selection command to be displayed on the patient terminal 2 via the network N as the message from the supporter.

As described above, in the present embodiment, only the messages effective for the treatment of the patient with MCI are prepared in advance in the message DB 422 under the supervision of the experts who have relevant knowledge of the treatment of the patient with MCI. Thus, the supporter can autonomously and safely interact with the patient to effectively support the patient by selecting, from the message DB 422, the message for supporting the patient who is playing the game. This can prevent the deterioration of the symptoms of the patient with MCI. This also allows early detection of symptoms in the patient with MCI to handle them. Furthermore, the mental and physical health of the supporter is improved by providing "psychological support" such as reducing the stress of the supporter (family, etc.) other than the patient. As a result, it is also possible to contribute to the improvement of the therapeutic effect of the patient himself/herself.

The present invention is not limited to the above-described embodiment, and various modifications and applications are possible. Modifications of the above-described embodiment applicable to the present invention will be described below.

In the above embodiment, MCI is exemplified as the treatment target. However, the present invention is not limited thereto, and the treatment target is arbitrary as long as the patient can perform the training for the treatment by using the patient terminal 2. The treatment target may be the dementia, the developmental disorder, the gambling addiction, or the like. Alternatively, the treatment target may be the hypertension, the chronic pain, the internal medical disease such as cancer, or the like.

In the above-described embodiment, the programs executed by the CPU each of the control units 24, 34, and 43 are described as being stored in advance in the ROM, the storage units 21, 31, and 42, and the like, but the present invention is not limited thereto. The programs for executing the above-described processes may be applied to conventional general-purpose computers to function as the patient terminal 2, the supporter terminal 3-n, and the server device 4 according to the above-described embodiment.

The method for providing such programs is arbitrary. For example, the programs may be stored in a computer-readable recording medium (flexible disk, CD (Compact Disc)-ROM, DVD (Digital Versatile Disc)-ROM, etc.), and distributed. Further, the programs may be stored in a storage on a network such as the Internet, and provided by downloading the programs.

Further, when the above processes are executed by sharing the responsibility between an OS (Operating System) with an application program or by cooperation with the OS and the application program, only the application program may be stored in the recording medium or the storage. It is also possible to superimpose a program on a carrier wave and distribute it via a network. For example, the program may be posted to a bulletin board system (BBS) on the network, and the program may be distributed via the network. Then, the above-described processes may be executed by starting the program and executing the program in the same manner as the other application programs under the control of the OS.

It should be noted that the present invention is capable of various embodiments and modifications without departing from the broadly defined spirit and the scope of the present invention. Also, the above-described embodiment is intended to illustrate one embodiment of the present invention and is not intended to limit the scope of the present invention.

### EXPLANATION OF REFERENCE NUMERALS

1: Treatment support system
2: Patient terminal
3-n: Supporter terminal
4: Server device
21,31,42: Storage unit
22,32: Touch panel
23,33,41: Communication unit
24,34,43: Control unit
300: Play screen
421: Patient DB
422: Message DB
423: Usage log DB
424: Mental log DB
500: Message selection screen

## Claims

1. A server device (4) connected to a patient terminal (2) used by a patient for training for treatment and a supporter terminal (3-n) of a supporter of the patient via a network (N), the server device (4) comprising:
a message database (422) which registers messages effective for the treatment of the patient; and
a server control unit (43) wherein when a predetermined condition is satisfied, the server control unit (43) causes the messages registered in the message database (422) to be displayed on the supporter terminal (3-n) via the network (N) in a selectable manner, and wherein the server control unit (43) causes a message selected by the supporter with the supporter terminal (3-n) to be displayed on the patient terminal (2) via the network (N) as a message from the supporter.

2. The server device (4) according to claim 1, wherein in response to receiving a usage history of the patient terminal (2) via the network (N), the server control unit (43) determines whether or not a disengagement sign from the training is recognized in the patient based on the usage history of the patient terminal (2), and
wherein if the server control unit (43) determines that the disengagement sign is recognized in the patient, assuming that the predetermined condition is satisfied, the server control unit (43) causes the messages registered in the message database (422) to be displayed on the supporter terminal (3-n) via the network (N) in a selectable manner.

3. The server device (4) according to claim 2, wherein the server control unit (43) specifies a frequency of use of a treatment program for treating the patient based on the usage history of the patient terminal (2), the treatment program installed in the patient terminal (2), and
wherein if the frequency of use of the treatment program does not satisfy a predetermined standard, the server control unit (43) determines that the disengagement sign from the training is recognized in the patient.

4. The server device (4) according to claim 2, wherein the server control unit (43) specifies a degree of achievement of a treatment menu of the patient based on the usage history of the patient terminal (2), and
wherein if the degree of achievement of the treatment menu does not satisfy a predetermined standard, the server control unit (43) determines that the disengagement sign from the training is recognized in the patient.

5. The server device (4) according to claim 3, wherein if the frequency of use of the treatment program satisfies the predetermined standard, the server control unit (43) specifies a degree of achievement of a treatment menu of the patient based on the usage history of the patient terminal (2), and
wherein if the frequency of use of the treatment program satisfies the predetermined standard, but the degree of achievement of the treatment menu does not satisfy the predetermined standard, the server control unit (43) determines that the disengagement sign from the training is recognized in the patient.

6. The server device (4) according to claim 1, wherein in response to receiving an answer to a questionnaire about a lifestyle habit via the network (N), the answer input by the patient into the patient terminal (2), the server control unit (43) determines whether or not a disturbance of the lifestyle habit is recognized in the patient from the answer to the questionnaire about the lifestyle habit, and
wherein if the server control unit (43) determines that the disturbance of the lifestyle habit is recognized in the patient, assuming that the predetermined condition is satisfied, the server control unit (43) causes the messages registered in the message database (422) to be displayed on the supporter terminal (3-n) via the network (N) in a selectable manner.

7. The server device (4) according to claim 6, wherein in response to receiving a usage history of the patient terminal (2) via the network (N), the server control unit (43) determines whether or not a disengagement sign from the training is recognized in the patient based on the usage history of the patient terminal (2), and
wherein if the server control unit (43) determines that the disengagement sign is recognized in the patient, the server control unit (43) determines whether or not the disturbance of the lifestyle habit is recognized therein.

8. The server device (4) according to claim 1, wherein in response to receiving an answer to a questionnaire about a patient's mood via the network (N), the answer input by the patient into the patient terminal (2), the server control unit (43) determines whether or not a deterioration of a mental state is recognized in the patient from a viewpoint of the patient based on the answer to the questionnaire about the patient's mood, and
wherein if the server control unit (43) determines that the deterioration of the mental state is recognized in the patient from the viewpoint of the patient, assuming that the predetermined condition is satisfied, the server control unit (43) causes the messages registered in the message database (422) to be displayed on the supporter terminal (3-n) via the network (N) in a selectable manner.

9. The server device (4) according to claim 8, wherein in response to receiving an answer to a questionnaire about the patient's mood via the network (N), the answer input by the supporter into the supporter terminal (3-n), the server control unit (43) determines whether or not the deterioration of the mental state is recognized in the patient from a viewpoint of the supporter based on the answer to the questionnaire about the patient's mood, and
wherein if the server control unit (43) determines that the deterioration of the mental state is recognized in the patient from the viewpoint of the patient or the viewpoint of the supporter, assuming that the predetermined condition is satisfied, the server control unit (43) causes the messages registered in the message database (422) to be displayed on the supporter terminal (3-n) via the network (N) in a selectable manner.

10. The server device (4) according to claim 1, wherein in response to receiving a usage history of the patient terminal (2) and an answer to a questionnaire about a lifestyle habit via the network (N), the answer input by the patient into the patient terminal (2), the server control unit (43) specifies a degree of achievement of a treatment menu of the patient based on the usage history of the patient terminal (2), and specifies a degree of achievement of the lifestyle habit of the patient from the answer to the questionnaire about the lifestyle habit, and
wherein if both the treatment menu and the lifestyle habit satisfy a predetermined standard, the server control unit (43) causes messages praising the patient among the messages registered in the message database (422) to be displayed on the supporter terminal (3-n) via the network (N) in a selectable mannered, or if the treatment menu or the lifestyle habit does not satisfy the predetermined standard, the server control unit (43) causes messages for supporting the patient among the messages registered in the message database (422) to be displayed on the supporter terminal (3-n) via the network (N) in a selectable manner.

11. The server device (4) according to claim 1, wherein in response to receiving an answer to a questionnaire about a treatment activity via the network (N), the answer input by the patient into the patient terminal (2), the server control unit (43) determines whether or not a disengagement sign from the training is recognized in the patient from the answer to the questionnaire about the treatment activity, and
wherein if the server control unit (43) determines that the disengagement sign is recognized in the patient, assuming that the predetermined condition is satisfied, the server control unit (43) causes the messages registered in the message database (422) to be displayed on the supporter terminal (3-n) via the network (N) in a selectable manner.

12. A treatment support system (1) comprising: a server device (4) connected to a patient terminal (2) used by a patient for training for treatment via a network (N); and a supporter terminal (3-n) of a supporter of the patient connected to the server device (4) via the network (N),
wherein the server device (4) comprises:
a message database (422) which registers messages effective for the treatment of the patient; and
a server control unit (43) wherein when a predetermined condition is satisfied, the server control unit (43) transmits display data for displaying the messages registered in the message database (422) in a selectable manner to the supporter terminal (3-n) via the network (N), and wherein in response to receiving a message selection command transmitted from the supporter terminal (3-n) via the network (N), the server control unit (43) causes a message specified from the message selection command to be displayed on the patient terminal (2) via the network (N) as a message from the supporter,
wherein the supporter terminal (3-n) comprises:
a display unit (32) wherein in response to receiving the display data transmitted from the server device (4) via the network (N), the display unit (32) displays the messages effective for the treatment of the patient in a selectable manner based on the display data; and
a terminal control unit (34) which transmits the message selection command indicating a message selected by the supporter from among the messages displayed on the display unit (32) to the server device (4) via the network (N).

13. A treatment support method with a server device (4) connected to a patient terminal (2) used by a patient for training for treatment and a supporter terminal (3-n) of a supporter of the patient via a network (N), the server device (4) having a message database (422) for registering messages effective for the treatment of the patient, the treatment support method comprising:
displaying the messages registered in the message database (422) on the supporter terminal (3-n) via the network (N) in a selectable manner, when a predetermined condition is satisfied; and
displaying a message selected by the supporter with the supporter terminal (3-n) on the patient terminal (2) via the network (N) as a message from the supporter.

14. A treatment support method in a treatment support system (1) including: a server device (4) connected to a patient terminal (2) used by a patient for training for treatment via a network (N) and having a message database (422) for registering messages effective for the treatment of the patient; and a supporter terminal (3-n) of a supporter of the patient connected to the server device (4) via the network (N),
wherein when a predetermined condition is satisfied, the server device (4) transmits display data for displaying the messages registered in the message database (422) in a selectable manner to the supporter terminal (3-n) via the network (N),
wherein in response to receiving the display data transmitted from the server device (4) via the network (N), the supporter terminal (3-n) displays the messages registered in the message database (422) on a display unit (32) in a selectable manner based on the display data,
wherein the supporter terminal (3-n) transmits a message selection command indicating a message selected by the supporter from among the messages displayed on the display unit (32) to the server device (4) via the network (N), and
wherein in response to receiving the message selection command transmitted from the supporter terminal (3-n) via the network (N), the server device (4) causes the message specified from the message selection command to be displayed on the patient terminal (2) via the network (N) as a message from the supporter.

15. A program for causing a computer of a server device (4), which is connected to a patient terminal (2) used by a patient for training for treatment and a supporter terminal (3-n) of a supporter of the patient via a network (N), and includes a message database (422) for registering messages effective for the treatment of the patient, to execute
a process for displaying the messages registered in the message database (422) on the supporter terminal (3-n) via the network (N) in a selectable manner, when a predetermined condition is satisfied; and
a process for displaying a message selected by the supporter with the supporter terminal (3-n) on the patient terminal (2) via the network (N) as a message from the supporter.
